# EUROPEAN PATENT APPLICATION

(11) **EP 2 596 750 A1**
(43) Date of publication of application: **29.05.2013**
(21) Application number: 11868523.9
(22) Date of filing: 30.06.2011
(51) Int. Cl.: A61B 6/03

(54) **X-RAY DIAGNOSTIC DEVICE**

(71) Applicant: Kanzaki Clinic of Cardiovascular Medicine Medical Corporation, Oita 870-0021 (JP)
(72) Inventor: KANZAKI Koreyasu, Oita-shi Oita 870-0021 (JP)
(74) Representative: Hössle Patentanwälte Partnerschaft
(86) International application number: PCT/JP2011/065011
(87) International publication number: WO 2013/001635

(57) **Abstract**

A recorded image of moving images substantially synchronized to pulsation of a heart are superimposed on an X-ray image taken during a medical procedure and are together displayed in composite as a three-dimensional image on a 3D display. Safety of a coronary artery intervention and catheterization is thus improved and increased procedural efficiency can be realized.

## Description

### Technical Field

The present invention relates to an X-ray imaging apparatus; specifically, an X-ray diagnosis apparatus which improves safety and efficiency for an intravascular procedure (intervention) using a catheter by enabling X-ray images and recorded moving images taken during a medical procedure to be viewed in three-dimensional images.

### Background Art

A conventional X-ray image capturer in an X-ray diagnosis apparatus is configured so as to capture an image of a treatment portion (focus area) of a patient (test subject) by providing one X-ray tube and one X-ray detector facing each other on two end portions of a support arm. In the X-ray diagnosis apparatus, an X-ray image is displayed as a two-dimensional image and is video recorded as a two-dimensional image. Therefore, when capturing an image of a blood vessel having many branches, as in the coronary artery of the heart, it has been necessary to inject a contrast medium into the blood vessel and capture images of the treatment portion from many directions. Thus, the amount of contrast medium used increases and a great physical burden has been placed on the patient.

In a conventional X-ray diagnosis apparatus of this kind, when performing a coronary artery intervention (a type of catheterization), a doctor has needed a two-dimensional image display device to display a video image that was captured from the same direction as the X-ray image, from among a plurality of video images captured in advance from a plurality of directions. The doctor has then needed to perform guide wire manipulation in the X-ray image while referring to the recorded image and, each time a branch portion branching off in a complex way is reached, has needed to inject contrast medium and take an angiogram to confirm the direction in which the guide wire is proceeding.

Were the doctor able to display a stenotic lesion in a complex coronary artery branch having many branches with a three-dimensional image, the doctor would be able to stereoscopically visualize the coronary artery branch and the site of stenosis. Therefore, development of an X-ray diagnosis apparatus having three-dimensional image display functionality has been long awaited. The disclosures of Patent Documents 1 to 3 have been developed recently as X-ray diagnosis apparatuses of this kind.

The X-ray diagnosis apparatus of Patent Document 1 (Japanese Unexamined Patent Publication No. 2010-194046) creates a road map of a focus area from data in a three-dimensional vascular image of a vascular region in a patient that was recorded in the past, then displays the road map in composite with a live image of the treatment portion (an X-ray image taken during a medical procedure). Moreover, position information for the focus area in the three-dimensional image is recorded by a recorder. Based on that position information, when a viewing angle is changed during the medical procedure, the three-dimensional image of the road map is also changed so as to substantially match the changed angle. The X-ray diagnosis apparatus of Patent Document 2 (Japanese Unexamined Patent Publication No. 2010-115481) reconstructs data for a three-dimensional image of an object of interest based on vascular image data captured from many directions and three-dimensional coordinates obtained from the capture directions, then displays an image that facilitates viewing of the object of interest on a two-dimensional image display apparatus. The X-ray diagnosis apparatus of Patent Document 3 (Japanese Unexamined Patent Publication No. 2009-213892) reconstructs a three-dimensional image from data captured using a robot arm during a medical procedure, then displays a three-dimensional image on a two-dimensional display.

When a plurality of recorded images having different temporal axes can be displayed simultaneously during a coronary artery intervention, completely occluded coronary artery branches and blood vessels peripheral to the occluded portions, which have received contrast medium due to collateral circulation, can be displayed on the same screen and provide a useful reference image for the doctor. The disclosures of Patent Documents 4 and 5, for example, are known as conventional art capable of providing images of this kind. The X-ray diagnosis apparatus of Patent Document 4 (Japanese Unexamined Patent Publication No. 2008-125616) defines a plurality of display configuration conditions for displaying an image of a test subject, then creates from four-dimensional image data of the test subject that includes a temporal element a corresponding plurality of three-dimensional image data that includes a temporal element for each of the plurality of display configuration conditions. When the plurality of display configuration conditions have been fixed, the X-ray diagnosis apparatus of Patent Document 4 then switches between the three-dimensional image data for each of the fixed display configuration conditions and displays a moving image on a monitor screen. Thereby, images can be displayed in which a most appropriate viewing direction has been determined in a short amount of time.

The X-ray diagnosis apparatus of Patent Document 5 (Japanese Unexamined Patent Publicaiotion No. 2010-148862) includes a four-dimensional image data generator employing image information obtained by capturing an image of a test subject to generate four-dimensional image data configured with a plurality of three-dimensional image data having information that indicates an order for each on a temporal axis, and an image generator generating moving image data configured with a plurality of two-dimensional image data generated from the three-dimensional image data that configures the four-dimensional image data and generating association information in which each of the two-dimensional image data configuring the moving image data is associated with the three-dimensional image data that serves as a source. Thereby, regardless of the capacities of the display apparatus employed, image data having a large amount of data can be displayed with simple operations.

### Summary of the Invention

However, even when creating a three-dimensional image from vascular image capture data employing any of the X-ray diagnosis apparatuses disclosed in Patent Documents 1 to 3, the reference three-dimensional image is a three-dimensional computer graphics (3D CG) image in which a target having three-point coordinates is perspective-projected on an imaginary two-dimensional coordinate screen. An X-ray image taken during a medical procedure remains a two-dimensional image, and thus there is a divergence in visibility between the X-ray image and the 3D CG. When a doctor changes a direction of a fluoroscope, the X-ray image and the three-dimensional image of the same direction must be extracted from a large amount of image data, and thus a time lag arises when displaying on a screen, extending a procedure's duration.

Moreover, in a coronary artery intervention, a periphery to an occluded site cannot be seen, and thus feeding guide wires to a totally occluded lesion is a difficult task. Were the occluded coronary artery branch and the periphery to the occluded site, which has received contrast medium due to collateral circulation, able to be displayed on the same screen, this would be useful, and were a four-dimensional image display apparatus such as, e.g., in Patent Documents 4 and 5 employed, there is a possibility that two three-dimensional recorded images having different temporal axes could be displayed on the same screen. However, even in a case where the conventional apparatuses are employed, the three-dimensional image constructed is displayed on a screen for two-dimensions and X-ray images taken during the medical procedure remain as two-dimensional images.

As a result of committed study, the inventors have devised to convert X-ray images taken during the medical procedure (live images) and recorded images of moving images substantially matched to a heartbeat into respective three-dimensional images. The obtained three-dimensional X-ray images taken during the medical procedure and the three-dimensional recorded images of the moving images are superimposed in a composite display on a 3D display screen. A stereoscopic view of how a catheter or guide wire is moving through a blood vessel or heart is then enabled. Observing that, as a result, a level of safety increases for a catheterization such as a coronary artery intervention and efficiency of the procedure also increases, the present invention was completed. The present invention has as an object to provide an X-ray diagnosis apparatus capable of improving the level of safety for an intravascular operation using a catheter as well as the efficiency of the procedure.

### Means for Solving the Problems

The present invention according to claim 1 is an X-ray diagnosis apparatus that includes an X-ray image capturer capturing three-dimensional X-ray images of a focus area on a test subject and a three-dimensional image processor obtaining three-dimensional moving images of the focus area which are used during a catheterization of the focus area based on X-ray image data captured by the X-ray image capturer. The X-ray image capturer includes: two X-ray tubes projecting X-ray beams from two different directions toward the test subject atop a platform table; one or two X-ray detector(s) obtaining X-ray image data of the test subject from the two different directions by detecting the X-ray beams projected from the two X-ray tubes; an image capture angle changing means changing an image capture angle of the test subject when X-ray images of the focus area on the test subject are captured using the two X-ray tubes and the one or two X-ray detector(s); and an image capture angle detecting means detecting the image capture angle of the test subject when X-ray images of the focus area on the test subject are captured using the two X-ray tubes and the one or two X-ray detector(s). The three-dimensional image processor includes: a three-dimensional image composer composing three-dimensional image data by correcting with digital image processing center-offset in an area of interest in X-ray image data of the test subject from the two different directions that was detected by the one or two X-ray detector(s) and a distortion of the X-ray image data of the test subject from the two different directions; a memory in which the three-dimensional image data composed by the three-dimensional image composer and vascular image data of the focus area where a contrast medium has been injected are each associated and stored with electrocardiogram data and image capture angle data from the image capture angle detecting means taken during X-ray image capture of the test subject; a moving image memory storing three-dimensional vascular moving image data for at least one heartbeat that was generated by associating each of the electrocardiogram data and the image capture angle data from the image capture angle detecting means taken during X-ray image capture of the test subject with the vascular image data for at least one heartbeat from the vascular image data of the focus area stored in the memory; a system controller changing the image capture angle of the test subject with the image capture angle changing means such that the image capture angle is the same as the image capture angle data associated with the three-dimensional vascular moving image data stored in the moving image memory; a 3D display stereoscopically displaying a three-dimensional vascular moving image for at least one heartbeat that was generated based on the three-dimensional vascular moving image data for at least one heartbeat stored in the moving image memory and a three-dimensional X-ray image obtained by capturing an X-ray image of the focus area during the medical procedure with the X-ray image capturer from the two different directions, such that each stands out from a screen; and a three-dimensional image compositer in which the three-dimensional vascular moving image for at least one heartbeat (stored in the moving image memory) is synchronized to an electrocardiogram of the test subject during the medical procedure and is repeatedly replayed on a screen of the 3D display and, after changing the image capture angle of the test subject with the system controller, the three-dimensional vascular moving image for at least one heartbeat that is being repeatedly replayed and the three-dimensional X-ray images of the focus area taken during the medical procedure are substantially synchronized and stereoscopically displayed superimposed on the screen of the 3D display.

Examples of the focus area diagnosable by the X-ray diagnosis apparatus include a coronary artery, a cardiac ventricle, a cardiac atrium, an atrial septum, a ventricular septum, a heart valve, a pulmonary artery, an aorta, and the like in the test subject's heart, and vascular channels throughout the body, such as cerebral blood vessels and a hepatic artery. The two X-ray tubes (X-ray tube bulbs) are powered by a high-voltage generator and also serve to obtain left-eye X-ray image data and right-eye X-ray image data. The bidirectional X-ray beams projected from the two X-ray tubes are projected onto an X-ray detection surface of the one or two X-ray detector(s) after intersecting at the focus area of the test subject, thereby obtaining shadow images for the left eye and the right eye that have passed through the test subject. Accordingly, the bidirectional X-ray image data obtained from the X-ray detector(s) is two-dimensional (flat) X-ray image data for the left eye and the right eye, which is required in order to obtain a stereoscopic X-ray image. With this method, X-ray images of the test subject can be captured while changing the image capture angle and several groups of bidirectional X-ray image data can be obtained.

In a case configured such that one X-ray detector is provided facing the two X-ray tubes, power is supplied alternatingly to the two X-ray tubes from the high-voltage generator. Thus, a circumstance is resolved in which X-ray beams from the two X-ray tubes project onto the single X-ray detector simultaneously, making it impossible to obtain an X-ray image. In a case where two X-ray detectors are employed, the two X-ray beams projected from each of the X-ray tubes project onto the two X-ray detectors respectively. Thus, power is supplied constantly to the two X-ray tubes from the high-voltage generator, and X-ray beams can be projected continuously from the two X-ray tubes. Examples of the image capture angle changing means which may be employed include a rotator rotating a support arm, which is provided with the X-ray tubes and the X-ray detector(s) facing each other at both ends thereof in a length direction, around the platform table; a driver causing the X-ray detector to move at an end of the support arm; and a platform table displacer which can slide the platform table forward, backward, right, and left, and can change the height of the platform table. The platform table can be operated by an operating portion in a control room; however, when necessary, the doctor can cause forward, backward, right, and left displacement with a manual lever attached to the platform table, and can change the height of the platform table with a foot pedal.

A projection angle of the two X-ray beams projected from the two X-ray tubes can be altered with an X-ray tube displacer capable of changing a distance between the two X-ray tubes. However, the X-ray tube displacer can also be considered a kind of image capture angle changing means. Herein, the "image capture angle" means the projection angle of the X-ray beams when the X-ray beams are projected onto the test subject to obtain an X-ray shadow image. When the image capture angle is greatly changed, for example, the X-ray tubes and the X-ray detector(s) are rotated around the test subject as a single set, or the platform bed to which the test subject has been fixed is rotated between the X-ray tubes and the X-ray detector(s). Moreover, an "X-ray beam angle" means the projection angle of the X-ray beams changed by differing the separation distance of the two X-ray tubes. Examples of the image capture angle detecting means which may be employed include various types of angle sensors, position sensors, and the like.

"Three-dimensional image data and vascular image data of the focus area are each associated with electrocardiogram data and image capture angle data from the image capture angle detecting means taken during X-ray image capture of the test subject" means that when the three-dimensional image data and the vascular image data of the focus area are stored in the memory, the image capture angle data (which has been changed by the image capture angle changing means while corresponding image data was obtained and which has been detected by the image capture angle detecting means) and the electrocardiogram data of the test subject (taken while corresponding image data was obtained) are stored in the memory integrally (as one block) with the respective image data. An R-wave is best for the electrocardiogram employed for synchronization to substantially match movement of the heart in the recorded moving image and the live moving image at systole and diastole. However, depending on necessity, other waveforms may be employed.

By greatly changing the image capture angle, several groups of bidirectional X-ray image data can be obtained. Herein, "several groups of bidirectional X-ray image data" means X-ray image data obtained by changing the image capture angle for one group of left-eye X-ray image data and right-eye X-ray image data. In order to obtain the vascular image data, a DSA procedure, for example, is carried out with respect to the focus area where a contrast medium has been injected. DSA procedure stands for Digital Subtraction Angiography procedure. Specifically, this is a method in which a mask image of the focus area on the test subject is first captured prior to injection of the contrast medium, then a contrast image of the focus area is captured by injecting the contrast medium into the test subject. Next, a subtraction process is performed on the mask image and the contrast image to remove background such as bone and obtain an image where only blood vessels remain (a DSA image). The "vascular image data for at least one heartbeat" is the vascular image data obtained while the heart pulses one time or two or more times. For example, in a case of one heartbeat over one second, the left-eye and right-eye vascular image data will be between 15 and 30 frames per second for each. Moreover, the "three-dimensional vascular moving image data for at least one heartbeat," the "X-ray image data for at least one heartbeat," and the like have simply changed the vascular image data to three-dimensional vascular moving image data or X-ray image data or the like, and have a similar meaning.

A type of the 3D display is subject to discretion. For example, 3D displays can be employed such as a frame sequential method requiring liquid crystal shutter glasses, a polarized film method not requiring the liquid crystal shutter glasses, or a lenticular lens method capable of naked-eye support. The three-dimensional X-ray image and the three-dimensional vascular moving image on the 3D display screen may be displayed in a uniform color or in different colors.

In particular, the present invention according to claim 2 is the X-ray diagnosis apparatus according to claim 1 in which the X-ray image capturer includes: a support arm on which two X-ray tubes are disposed at one end in a length direction and one X-ray detector is provided at a second end in the length direction facing the two X-ray tubes; and a high-voltage generator supplying power alternatingly to the two X-ray tubes such that the X-ray beams from the two X-ray tubes are not simultaneously projected onto the single X-ray detector. The image capture angle changing means rotates the support arm around a rotation shaft orthogonal to the length direction of the support arm and displaces the support arm in the length direction thereof.

The present invention according to claim 3 is the X-ray diagnosis apparatus according to claim 1 or claim 2 in which there is one X-ray detector and the X-ray detection surface thereof has a concave surface.

The present invention according to claim 4 is the X-ray diagnosis apparatus according to claim 1 including a three-dimensional exterior image composer generating two-dimensional image data from the two directions necessary in order to stereoscopically display on an exterior image display a three-dimensional exterior image of the focus area, which was generated based on three-dimensional exterior image data of the focus area on the test subject obtained from a second imaging diagnosis apparatus; and performing at least one of changing an image size of the three-dimensional exterior image, changing a viewing angle of the three-dimensional exterior image, viewing a cross-section at a predetermined position in the three-dimensional exterior image, and composite display of the three-dimensional exterior image on the 3D display screen superimposed on the three-dimensional vascular moving image stored in the moving image memory and the three-dimensional X-ray image taken during the medical procedure. An intracardiac ultrasound image captured during the medical procedure by an internal ultrasound imaging apparatus using an ultrasound catheter is viewed in comparison with the three-dimensional X-ray image taken during the medical procedure, is replayed as a moving image by recording captured three-dimensional X-ray images of the ultrasound catheter, and is displayed in composite with the three-dimensional X-ray images taken during the medical procedure.

The present invention according to claim 5 is the X-ray diagnosis apparatus according to claim 1 in which the number of X-ray detectors used is two, and the X-ray detectors and the two X-ray tubes are configured such that one of the X-ray detectors detects the X-ray beams projected from one of the X-ray tubes and a second X-ray detector detects the X-ray beams projected from a second X-ray tube.

The present invention according to claim 6 is the X-ray diagnosis apparatus according to claim 1 in which the X-ray image capturer includes two support arms on which one X-ray tube is provided at one end in the length direction and one X-ray detector is provided to a second end in the length direction facing the one X-ray tube; and in which the image capture angle changing means simultaneously rotates the two support arms in a uniform direction and to a uniform angle, and also simultaneously displaces the support arms a uniform distance to a uniform side in the length direction.

The present invention according to claim 7 is the X-ray diagnosis apparatus according to claim 1 in which the three-dimensional image compositer includes a three-dimensional image composer employing a color display as the 3D display, displaying the three-dimensional vascular moving images and the three-dimensional X-ray images in different colors on the 3D display screen, and additionally displaying blood vessels having different temporal axes in the three-dimensional vascular moving images on the 3D display screen in different colors.

### Effect of the Invention

According to the present invention, when a coronary artery intervention is performed, for example, X-ray beams are projected from two directions onto a test subject atop a platform table using two X-ray tubes, and a shadow image in which the X-ray beams have passed through the test subject is detected by one or two X-ray detector(s), thus obtaining X-ray image data for the test subject. The obtained bidirectional X-ray image data is associated with electrocardiogram data for at least one heartbeat and image capture angle data from an image capture angle detecting means. Next, each set of X-ray image data for at least one heartbeat, in which X-ray images of a focus area which has been injected with a contrast medium are captured from two different directions using the two X-ray tubes and the one or two X-ray detector(s), is DSA (Digital Subtraction Angiography) processed, for example. The vascular image data for at least one heartbeat thus obtained is associated with the electrocardiogram data and the image capture angle data taken while the X-ray image data was obtained to generate three-dimensional vascular moving image data for at least one heartbeat, which is then stored in a moving image memory.

Then, a command is sent from a system controller to an image capture angle changing means to change an image capture angle of the test subject so as to have the same image capture angle as the image capture angle data associated with the three-dimensional vascular moving image data for at least one heartbeat stored in the moving image memory. When the doctor views the X-ray image of the focus area on the test subject on the 3D display screen, the three-dimensional X-ray image taken during the medical procedure (hereafter referred to as live) is substantially synchronized (substantially matched) to the three-dimensional vascular moving image obtained from the three-dimensional vascular moving image data for at least one heartbeat stored in the moving image memory, and appears superimposed due to the three-dimensional image compositer. At this time, a vascular image is synchronized with the live electrocardiogram (e.g., an R-wave) to display at least one heartbeat in repeated replay as a three-dimensional recorded moving image. Thus, the doctor can visualize the way a guide wire is advancing through a blood vessel as a stereoscopic image rising up from the 3D display screen. Thereby, even when the guide wire inserted into the blood vessel has reached a branch in the vessel, for example, judging which direction a forefront portion of the guide wire that is bent in a "J" shape should be pointed is facilitated. Moreover, three-dimensional X-ray images can be captured even in a case where more three-dimensional vascular moving images in which the image capture angle is altered are added as necessary, reducing the amount of time before recording and playback.

Moreover, when a coronary artery intervention is performed on a coronary artery branch that is fully occluded, an image of the occluded site in the coronary artery branch and a periphery of the occluded site, which has received contrast medium by collateral circulation, is captured when the image capture angles for the test subject are in a uniform position by employing the image capture angle changing means ahead of image capture. Thereby, the occluded coronary artery branch and the blood vessels peripheral to the occluded site can be displayed in composite and, furthermore, these can be displayed in composite superimposed on the live three-dimensional X-ray image by a three-dimensional image compositer. Moreover, the live X-ray image and the recorded moving images displayed in composite are three-dimensional displays. The recorded image for at least one heartbeat is synchronized with the live electrocardiogram and is displayed in repeated replay on the 3D display screen; thus, the doctor can stereoscopically visualize how the guide wire is being inserted through an occluded site. As a result, judging which direction the forefront portion of the guide wire that is bent in a "J" shape should go to reach a blood vessel in a periphery beyond the occluded site is facilitated.

The present invention can also be applied to catheter ablation of an atrial fibrillation, for example. Specifically, in catheter ablation, a catheter for ablation must pierce an atrial septum using the Brockenbrough technique and be inserted into the left atrium. In advance of this procedure, left atrial contrasting is performed and three-dimensional images are recorded until the left atrium is contrasted by the contrast material from the right atrium via the right ventricle, pulmonary artery, and pulmonary vein. The obtained contrast images of the right atrium and the contrast images of the left atrium are, for example, displayed as three-dimensional images on the 3D display screen each in a different color. Then, by synchronizing with the live electrocardiogram, displaying the moving image for at least one heartbeat in repeated replay, and displaying in composite by superimposing the live X-ray image, the doctor can stereoscopically visualize a position in the atrial septum and an orientation of a forefront of a Brockenbrough needle that is bent in a "J" shape. Thereby, the doctor can safely and reliably pierce the atrial septum and, thereafter, the operation to push the forefront portion of the catheter for ablation to an appropriate site within the left atrium or proximal to the pulmonary vein can also be performed reliably and in a short amount of time.

As described above, according to the present invention, the live X-ray images and the recorded moving images are superimposed on the same 3D display and are displayed in composite three-dimensionally. Therefore, the way the guide wire or the catheter used during the medical procedure moves within a blood vessel or within the heart can be stereoscopically visualized in real time on the 3D display screen. Moreover, even when vascular contrast images have been recorded during the medical procedure due to necessity, immediate three-dimensional image replay display is possible and, when a catheterization treatment for the heart, such as a coronary artery intervention, is performed, the procedure can be made safer and can contribute to reducing the amount of time for the procedure.

In particular, according to the present invention according to claim 2, two X-ray tubes are provided on one support arm and one X-ray detector is provided at the other end in the length direction facing the two X-ray tubes. With such a configuration, when the high-voltage generator is provided alternatingly supplying power to the two X-ray tubes, X-ray beams from the two X-ray tubes will not be projected onto the single X-ray detector simultaneously. In addition, when the support arm is rotatable around a rotation shaft orthogonal to the length direction thereof and displaceable in the length direction thereof, at a time when the image capture angle of the test subject is changed, rotation by the image capture angle changing means to any angle centered on the test subject is enabled while maintaining a uniform distance between the two X-ray tubes and the one X-ray detector. Thereby, three-dimensional X-ray image capture and three-dimensional fluoroscopy from an angle required by the doctor are facilitated. Moreover, when attempting to obtain a good X-ray image, the distance between the X-ray detector and the test subject must be reduced for image capture; however, by having one X-ray detector, even when the distance between the test subject and the X-ray detector is extremely reduced for image capture, the distance between the two X-ray tubes does not increase and the two X-ray beams from right and left can reach the X-ray detector by passing through the region of interest on the test subject.

According to the present invention according to claim 3, the X-ray detector surface of the single X-ray detector has a concave shape facing the test subject and a length of the X-ray detector surface is configured to a length capable of detecting the bidirectional X-ray beams projected thereon alternatingly from the two X-ray tubes. Therefore, after intersecting at the focus area on the test subject, the X-ray beams projected from the two X-ray tubes arrive at the concave X-ray detector surface of the single X-ray detector at nearly orthogonal angles. As a result, distortion in the X-ray image of the test subject detected by the X-ray detector can be reduced as compared to a case where the X-ray detector surface is a flat surface. In addition, center-offset in an area of interest in the bidirectional X-ray images of the test subject detected by the X-ray detector can be corrected by the three-dimensional image composer of claim 1.

Moreover, according to the present invention according to claim 4, for example, past three-dimensional exterior image data obtained from a different imaging diagnosis apparatus is stored on an image server, and the required three-dimensional image data from among that data is loaded via an interface through a network line and stored in an exterior image memory as volume data. Computer image processing by the three-dimensional exterior image composer is performed thereon and two-dimensional image data from two directions for the left eye and the right eye is generated for stereoscopic display as a three-dimensional image on an exterior image display. At this point, by operating an operator such as a mouse while looking at the exterior image display, through computer digital image processing from the three-dimensional exterior image composer, the doctor performs at least one of changing an image size of the three-dimensional exterior image, changing a viewing angle of the three-dimensional exterior image, viewing a cross-section at a predetermined position in the three-dimensional exterior image, and composite display of the three-dimensional exterior image on the 3D display screen superimposed on the three-dimensional vascular moving image stored in the moving image memory and the three-dimensional X-ray image taken during the medical procedure. Thereby, the doctor can refer to the three-dimensional exterior image displayed stereoscopically on the exterior image display.

In addition, an intracardiac ultrasound image taken during the medical procedure with an internal ultrasound imaging apparatus using an ultrasound catheter is displayed as an image on the exterior image display via the interface, through the network line. Moreover, the orientation of the ultrasound catheter, which is bent in a "J" shape, is verified with the three-dimensional X-ray images taken during the medical procedure by superimposing the three-dimensional moving image on the 3D display with the composite display composer. They are then stored in a memory together with the electrocardiogram data and position information from the X-ray diagnosis apparatus displacer. The stored three-dimensional moving image of the ultrasound catheter is generated as a three-dimensional moving image for at least one heartbeat by a moving image selector, and the moving image and the three-dimensional X-ray image taken during the medical procedure are displayed in composite by the composite display composer in different colors on the 3D display.

According to the invention according to claim 5, two X-ray detectors and two X-ray tubes have a one-to-one relationship where one X-ray beam projected from one X-ray tube is detected by one X-ray detector and a second X-ray beam projected from a second X-ray tube is detected by a second X-ray detector. Thus, the X-ray beams can be projected onto the X-ray detector surfaces of the X-ray detectors head-on. Thereby, X-ray projection can be performed simultaneously from the two X-ray tubes without distortion arising in the X-ray images and without developing center-offset in the X-ray images.

According to the invention according to claim 6, two support arms are used in which one X-ray tube is provided at one end in a length direction and one X-ray detector is provided at a second end in the length direction. Thus, when changing an image capture angle, the two support arms are simultaneously rotated to the same angle in the same direction and are simultaneously displaced to the same distance on the same side of the length direction by the image capture angle changing means. Thereby, the X-ray beams can be projected onto the X-ray detector surfaces of the X-ray detectors head-on. Thus, X-ray projection can be performed simultaneously from the two X-ray tubes without distortion arising in the X-ray images and without developing center-offset in the X-ray images.

According to the invention according to claim 7, because the three-dimensional vascular moving image and the three-dimensional X-ray image are displayed in different colors on the screen of the 3D display, which is a color display, visibility of the three-dimensional vascular moving image and the three-dimensional X-ray image on the screen is facilitated. Moreover, by employing the color display, because the image compositing can be performed using the properties of the three elementary colors of light, composite images of shadow images of bones and soft organs do not become dark even when DSA processing is not performed. Specifically, in a conventional method using a display with a black and white screen, portions where shadows of bones and soft organs from the recorded image and the live X-ray image overlap become twice as dark and the shadows of the catheter and the guide wire may become difficult to see in the recorded vascular moving images and X-ray images. In contrast, in the case of the present invention, a color display is employed for the composite image display apparatus and when the recorded images, for example, are colored red and the live X-ray images, for example, are colored blue by the composite display composer, the bones and soft organs where the recorded images and the live X-ray images overlap become reddish-purple (magenta), the vascular images recorded with the vascular contrast are displayed in red, and the catheter and guide wire from the live X-ray images are displayed in blue. Moreover, these images are displayed on the 3D display, and so differences in distance (depth) are present and the shadows of bones and soft organs do not interfere when viewing the shadows of the vascular images, catheter, and guide wire.

In addition, when the catheter and guide wire are inserted into the blood vessels, superimposed on the vascular image, the guide wire in the vascular shadows (displayed in red) is displayed in reddish-purple. Moreover, there are various color combinations for the background color, which is not colored in the recorded images and the live X-ray images, such as remaining black or coloring a dark green, and these color combinations can be selected as desired with an operator. In a case where two images having different temporal axes must be superimposed for composite display, of the contrast images, for example, a left coronary artery image, when colored red, is displayed in red up to an occluded coronary artery branch; a periphery of the left coronary artery, which has received contrast from the right coronary artery via collateral circulation, is colored blue; and the live X-ray images of the catheter and guide wire are displayed in dark green. In such a case, the shadows of the bones and soft organs are displayed in light gray. Accordingly, the view is such that the guide wire (dark yellow) advances through the left coronary artery (red) and the guide wire (dark cyan) is fed through the occluded site in the blood vessel and proceeds into the peripheral blood vessel (blue). By employing the color display for the image display apparatus in this way, there is no further need for DSA processing in the three-dimensional moving image generator. As a result, capturing a background image for DSA processing becomes unnecessary. Therefore, operating the DSA processing in the three-dimensional moving image generator selects one heartbeat with the best contrast, then associates that image data with the electrocardiogram data and the position information data of the displacer for the X-ray image capture apparatus from when the image data was captured, which is then stored as data in the moving image memory. Thus, there is no need to perform operations to obtain a DSA processing background image nor to erase the background image from the contrast image based thereon in the image processing of the three-dimensional moving image generator. The operation of image processing is therefore simplified.

### Brief Description of the Drawings

Figure 1 is a block diagram showing a configuration of an X-ray diagnosis apparatus according to Example 1 of the present invention, in which a three-dimensional image processor is omitted.
Figure 2 is a block diagram showing a configuration of the three-dimensional image processor of the X-ray diagnosis apparatus according to Example 1 of the present invention.
Figure 3 is a perspective view of an X-ray image capturer configuring a portion of the X-ray diagnosis apparatus according to Example 1 of the present invention.
Figure 4 is a flow sheet showing a process during an X-ray diagnosis in Example 1 after composing a three-dimensional image of a test subject, up to storing X-ray image data in a first memory and displaying a three-dimensional X-ray image on a 3D display.
Figure 5 is an explanatory diagram showing a method for correcting center-offset in the X-ray images when X-ray beams from two X-ray tubes are projected at an angle onto a flat X-ray detector surface of a single X-ray detector during the X-ray diagnosis in Example 1.
Figure 6 is an explanatory diagram showing a difference in the X-ray images when the X-ray beams from the two X-ray tubes are projected onto the flat X-ray detector surface of the single X-ray detector head-on and when projected from an angle, and a method for correcting a distortion thereof during the X-ray diagnosis in Example 1.
Figure 7 is a perspective view of content in which the X-ray image data from two directions is associated with an electrocadiogram and stored in the first memory during the X-ray diagnosis with the X-ray diagnosis apparatus according to Example 1 of the present invention.
Figure 8 ia a flow sheet showing a first half of a process during the X-ray diagnosis in Example 1, up to DSA processing of the X-ray image data from two directions for each image capture angle that was stored in the first memory and storing of the three-dimensional vascular moving image obtained for each of the image capture angles in a second memory.
Figure 9 is a flow sheet showing a second half of a process during the X-ray diagnosis in Example 1, up to DSA processing of the X-ray image data from two directions for each image capture angle that was stored in the first memory and storing of the three-dimensional vascular moving image obtained for each of the image capture angles in the second memory.
Figure 10a is a perspective view showing the X-ray image data for one heartbeat from two directions associated with an elctroadiogram in which blood vessels are contrasted during the X-ray diagnosis in Example 1.
Figure 10b is a perspective view showing the X-ray image data for one heartbeat from two directions associated with the electrocadiogram before contrast of the blood vessels during the X-ray diagnosis in Example 1.
Figure 10c is a perspective view showing vascular moving image data for one heartbeat from two directions associated with the electrocadiogram after DSA processing during the X-ray diagnosis in Example 1.
Figure 11 is a flow sheet showing a first half of a process during the X-ray diagnosis in Example 1 after selecting required three-dimensional vascular moving image data from data in the second memory, up to displaying a composite of a three-dimensional X-ray image of the test subject taken during a medical procedure and the three-dimensional vascular moving image on a 3D display screen.
Figure 12 is a flow sheet showing a second half of a process during the X-ray diagnosis in Example 1 after selecting the required three-dimensional vascular moving image data from data in the second memory, up to displaying a composite of the three-dimensional X-ray image of the test subject taken during the medical procedure and the three-dimensional vascular moving image on the 3D display screen.
Figure 13 is a perspective view showing the vascular moving image data from two directions in a case where, during the X-ray diagnosis in Example 1, an identical number of heartbeats is associated with the three-dimensional vascular moving image data taken during the medical procedure and the three-dimensional vascular moving image data selected from the data in the second memory.
Figure 14 is a perspective view showing a processing method for vascular moving image data in a case where the number of heartbeats for the test subject during the medical procedure is faster than the number of heartbeats associated with the three-dimensional vascular moving image data selected from the data in the second memory during the X-ray diagnosis in Example 1.
Figure 15 is a perspective view showing a processing method for the vascular moving image data in a case where the number of heartbeats of the test subject during the medical procedure is slower than the number of heartbeats associated with the three-dimensional vascular moving image data selected from the data in the second memory.
Figure 16 is a flow sheet showing a process during the X-ray diagnosis in Example 1 where an occluded branch of a coronary artery and blood vessels on a periphery of the occluded site in a heart are stereoscopically displayed in composite on the 3D display screen to perform a catheterization treatment.
Figure 17 is a flow sheet showing a process during the X-ray diagnosis in Example 1, up to displaying a composite of a right atrial moving image obtained by right atrial contrasting and a left atrial moving image which is delayed in receiving the contrast medium on the 3D display in different colors and piercing an atrial septum with the Brockenbrough technique.
Figure 18 is a front view showing a state in which X-ray images of the test subject are captured using a single X-ray detector having a concave X-ray detector surface during an X-ray diagnosis that uses a different X-ray diagnosis apparatus according to Example 1 of the present invention.
Figure 19 is a perspective view of an X-ray diagnosis apparatus according to Example 2, in which two X-ray tubes and two X-ray detectors face each other on both ends of one support arm.
Figure 20 ia an enlarged lateral view of an essential portion of the X-ray diagnosis apparatus of Example 2.
Figure 21a is a perspective view of a different X-ray diagnosis apparatus of Example 2, in which two support arms are used on which one X-ray tube and one X-ray detector face each other on both ends.
Figure 21b is an exploded perspective view of an essential portion showing a configuration of a rotation portion of the two support arms in the different X-ray diagnosis apparatus of Example 2.
Figure 22 is a front view showing a state in which angles of two X-ray beams from the different X-ray diagnosis apparatus of Example 2 are narrowed and the X-ray detectors are distant from the test subject.
Figure 23 is a front view showing a state in which a distance between the two X-ray detectors and the test subject is narrowed in order to obtain a good-quality X-ray image during the X-ray diagnosis with the X-ray diagnosis apparatus of Example 2.
Figure 24 is a front view showing a state in which angles of the two X-ray beams from the X-ray diagnosis apparatus of Example 2 are narrowed and in which the X-ray detectors are distant from the test subject.

### Best Mode for Carrying out the Invention

Hereinafter, embodiments of the invention will be concretely described.

### Example 1

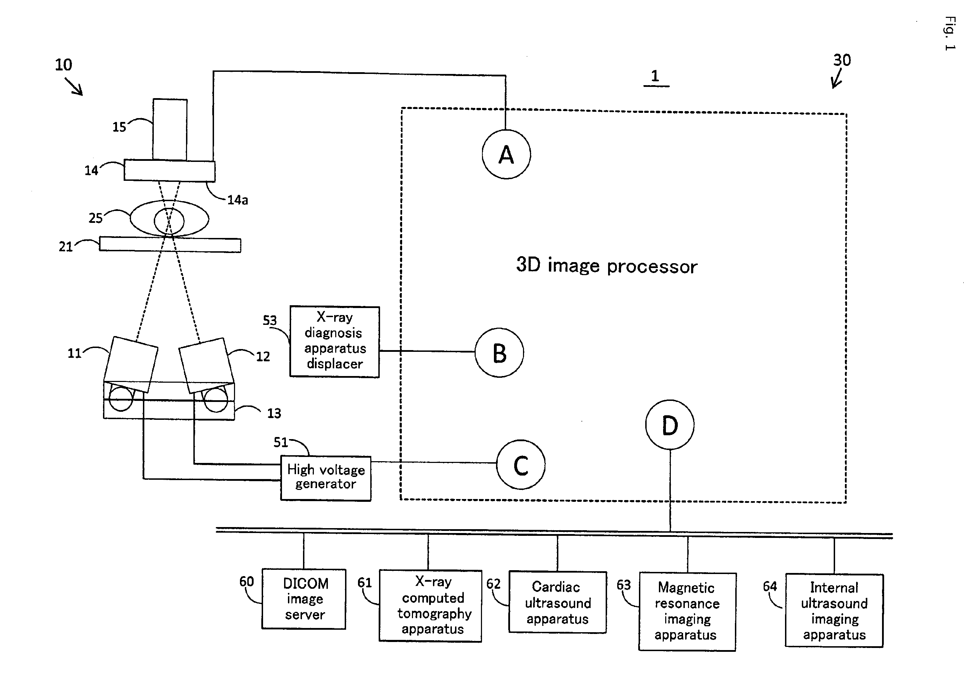

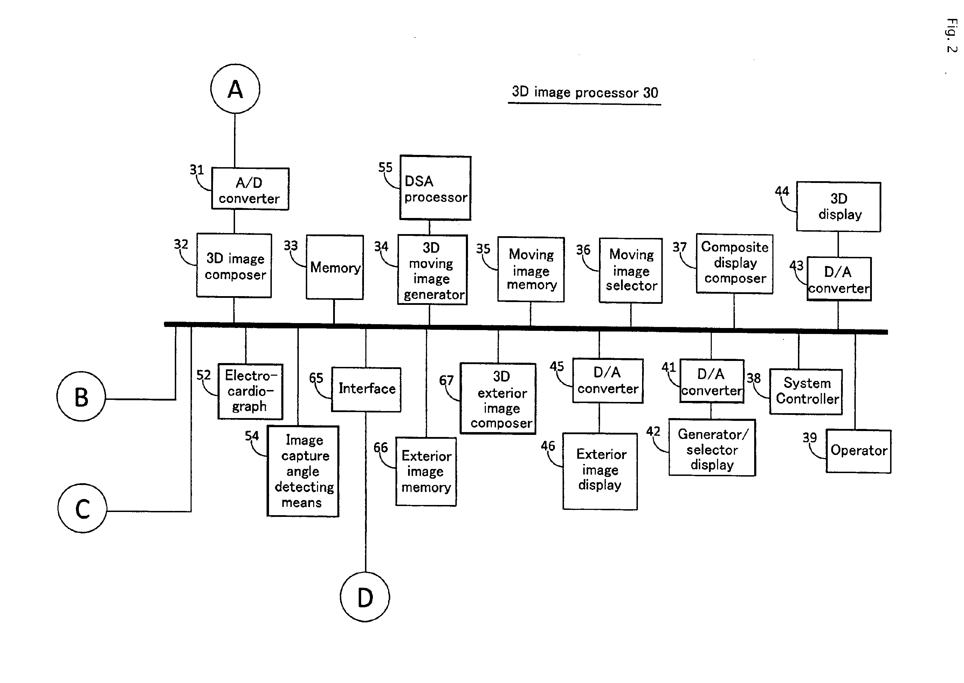

In Figures 1 and 2, an X-ray diagnosis apparatus 1 includes an X-ray image capturer 10 capturing X-ray images of a focus area on a test subject 25 and a three-dimensional image processor 30 obtaining, based on X-ray image data captured by the X-ray image capturer 10, three-dimensional moving images of the focus area required during a catheterization of the focus area.

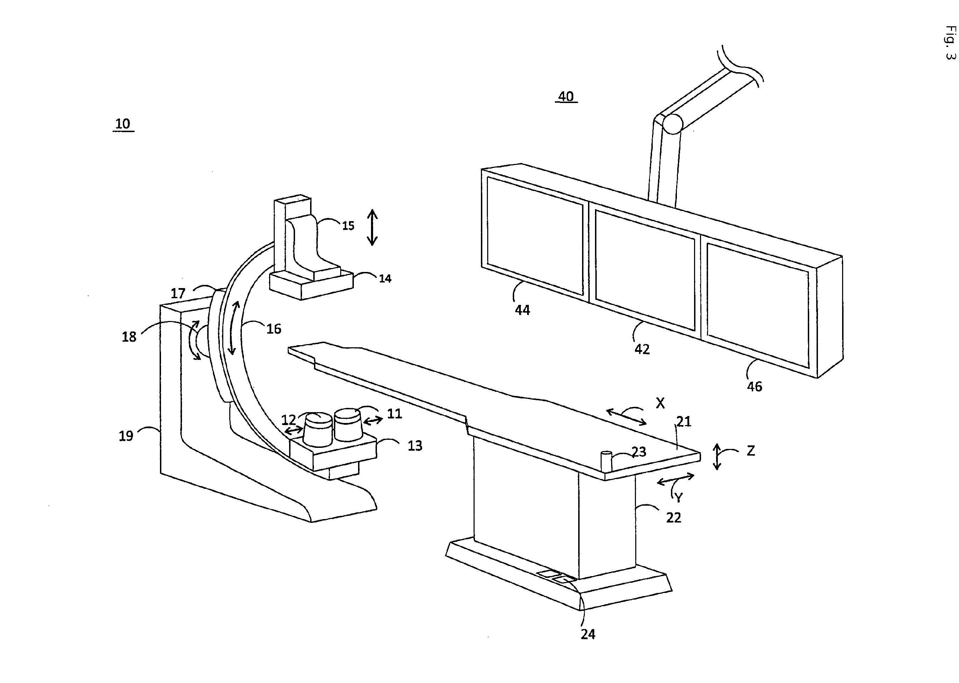

First, with reference to Figures 1 to 3, the X-ray image capturer 10 will be described in detail. The X-ray image capturer 10 includes one support arm 16; an X-ray diagnosis apparatus displacer (image capture angle changing means) 53 changing an image capture angle for the test subject 25 by displacing a movable portion that includes the support arm 16 in a predetermined direction during X-ray image capture of the test subject 25; an image capture angle detecting means 54 detecting an image capture angle of the test subject 25; two X-ray tubes 11 and 12 provided at one end of the support arm 16; and one X-ray detector 14 provided at a second end of the support arm 16.

The support arm 16 is a board material bending in a "C" shape that supports the X-ray tubes 11 and 12 and the X-ray detector 14 in an opposing state with the test subject 25 therebetween. The support arm 16 is provided to a top end portion of a vertical plate portion of a substantially L-shaped support stand 19 so as to be freely rotatable horizontally and freely slidable in an arm length direction. The X-ray diagnosis apparatus displacer 53 includes a rotation shaft 18 projecting horizontally toward a platform table 21 from the top end portion of the vertical plate portion of the support stand 19; a circular arc-shaped rotator 17 which is fixed to a foremost end of the rotation shaft 18 and has the support arm 16 mounted to be freely slidable in the length direction thereof; a driver 15 sliding the X-ray detector 14 on a support arm attachment mount fixed to the second end of the support arm 16 so as to freely approach/retreat with respect to the X-ray tubes 11 and 12; an X-ray tube displacer 13 changing a projection angle of X-ray beams from the two X-ray tubes 11 and 12; a compensating filter (not shown in the drawings) attached to the two X-ray tubes 11 and 12; and a platform table displacer 22 sliding a platform table 21 forward, backward, left, and right and changing a height of the platform table 21. Of these, the rotator 17 has as a main body a trench-shaped slide guide swept in a circular arc shape in which the support arm 16 is gripped so as to be freely slidable in the length direction thereof and includes, on an interior of the slide guide, a driver displacing the support arm 16 in the length direction thereof by rotating a feed roller with a feed motor. By rotating the feed roller in a predetermined direction with the feed motor, the support arm 16 slides in one direction or a second direction of the length direction thereof. In addition, at a command from a system controller 38 or an operator 39, the platform table 21 displaces at will in a length direction (X direction), a width direction (Y direction), or a vertical direction (Z direction) thereof with an X motor, Y motor, and Z motor installed therein. However, according to necessity, a doctor can adjust the platform table 21 forward, backward, left, and right using a hand lever 23 or a foot pedal 24.

The two X-ray tubes 11 and 12 alternatingly generate X-rays due to a high voltage alternatingly applied to each from a high voltage generator 51 at a rate of 15 to 30 times per second. The high voltage generator 51 generates the high voltage at a rate of 15 to 30 times per second, for example, at a command from the system controller 38. However, the doctor can adjust how many times per second the high voltage generation rate will be with a manual input operation from the operator 39, described hereafter. In addition, an angle of projected beams for the two X-ray tubes 11 and 12 can be changed with the X-ray tube displacer 13. The two X-ray tubes 11 and 12 may also be configured such that a distance between them can be widened and narrowed.

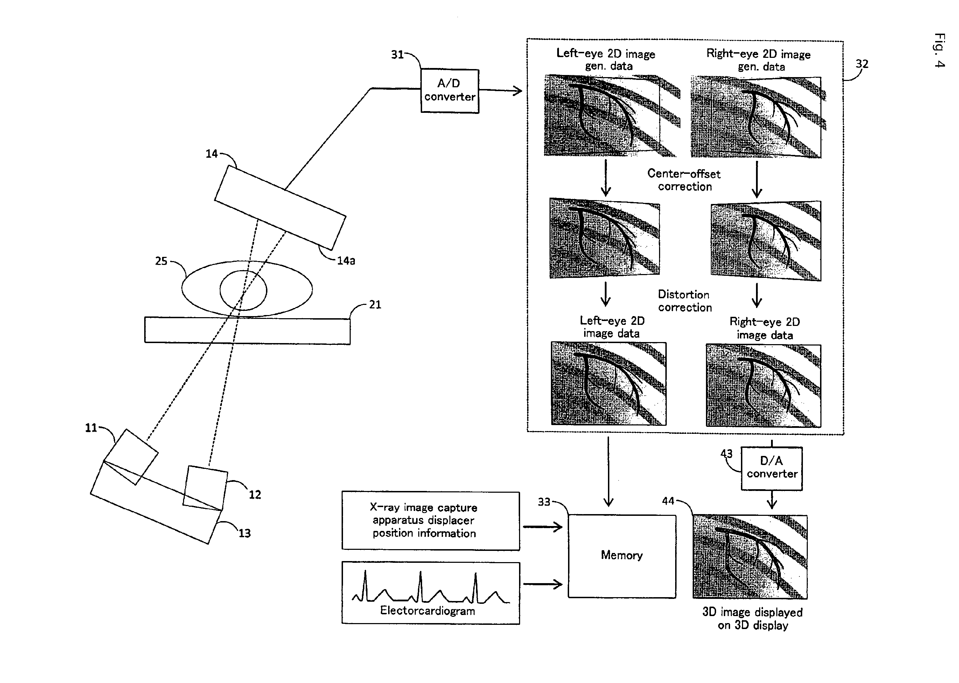

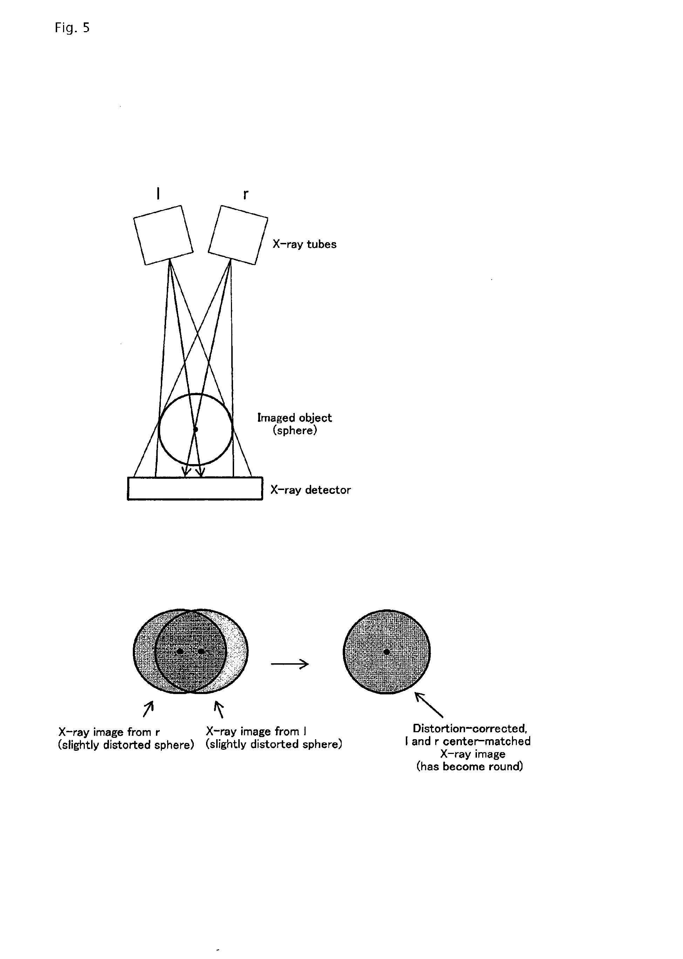

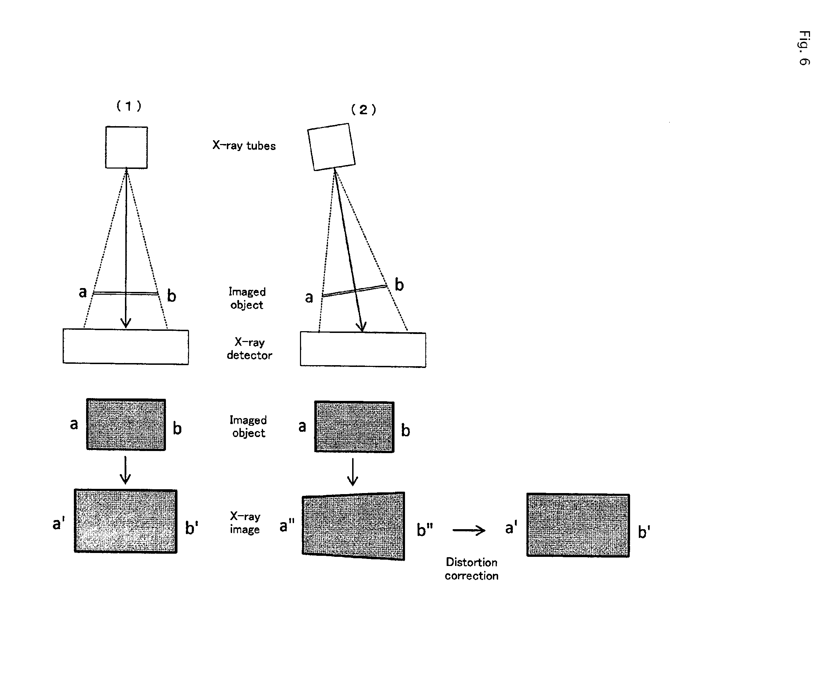

As shown in Figures 1 and 2, center lines of the two X-ray beams are adjusted, for example, to an angle of 10° each from the left and right directions toward the single X-ray detector 14 and intersect in a vicinity of the focus area of the test subject 25. When a projection angle from the left and right X-ray tubes 11 and 12 onto a flat X-ray detector surface 14a of the X-ray detector 14 is increased, a stereoscopic feel of the three-dimensional image is exceptional. However, when the doctor viewing the three-dimensional image stares at the screen for a long time, eye fatigue is likely to occur. Therefore, the doctor can output operation data from the operator 39, which configures a portion of the three-dimensional image processor 30, to make adjustments to reach an appropriate angle (here, 10°). In addition, distortion and center-offset in an image for the left eye and an image for the right eye occur in the X-ray image of the test subject 25 in proportion to the angle at which the X-ray beams project onto the X-ray detector surface 14a. Thus, as shown in Figure 4, the image distortion and center-offset in the live 2D (two-dimensional) image data obtained from the X-ray detector 14 can be corrected by digital processing in the three-dimensional image composer 32 and generated as digital image data similar to a case where the X-ray beams are projected head-on. The center-offset in the left and right images undergoes digital image correction with a method shown in Figure 5 and the distortion in the left and right images undergoes digital image correction with a method shown in Figure 6.

The image capture angle detecting means 54 includes sensors provided to each of the rotation shaft 18, the rotator 17, the driver 15, the X-ray tube displacer 13, the compensation filter, and the platform table displacer 22 of the X-ray diagnosis apparatus displacer 53, and performs detection with the sensors by digital signal conversion. A detection signal from the image capture angle detecting means 54 is sent to the memory 33 and is simultaneously transmitted to the system controller 38. The X-ray detector 14 detects the bidirectional X-ray beams that are generated alternatingly by the two X-ray tubes 11 and 12 and pass through the test subject 25. The X-ray detector 14 is configured with a flat panel detector (FPD) having a plurality of semiconductor detection elements arrayed in a matrix. Moreover, instead of the FPD, the X-ray detector 14 may also be configured with a combination of an image intensifier and a TV camera.

The three-dimensional image processor 30 includes an A/D converter 31; a three-dimensional image composer 32; a memory (first memory) 33; a three-dimensional moving image generator 34; a moving image memory (second memory) 35; a moving image selector 36; a composite display composer (three-dimensional image compositer) 37; the system controller 38; the operator 39; an image display apparatus 40; a generator/selector display 42; D/A converters 41, 43, and 45; a 3D display 44; an exterior image display 46; an electrocardiograph 52; a DSA processor 55; an interface 65; an exterior image memory 66; and a three-dimensional exterior image composer 67. Of these, the interface 65 is connected, via a network line, to a DICOM image server 60, an X-ray computed tomography apparatus 61, a cardiac ultrasound apparatus 62, a magnetic resonant imaging apparatus 63, and an internal ultrasound imaging apparatus 64. Moreover, each of the displays 42, 44, and 46 are three-dimensional-format (3D) color displays. In addition, an input image signal is displayed as a two-dimensional image when two-dimensional (2D) and is displayed as a three-dimensional image when three-dimensional.

The X-ray diagnosis apparatus displacer 53 of the X-ray diagnosis apparatus 1 is driven in response to operation data from the operator 39. All movement breadth (hereafter, position information) of the X-ray diagnosis apparatus displacer 53 during X-ray image capture is digitized. The left/right-differentiated two-dimensional X-ray image data (hereafter referred to at times as two-dimensional image data) is grouped with digital electrocadiogram data for each image capture and is then stored in the memory 33. In addition, when the X-ray image data is grouped at each X-ray image capture, a still image matched to an electrocardiograph R-wave for three heartbeats, for example, after X-ray image capture has begun is automatically stored in the memory 33 as image data. This is then made into a representative image when displaying in a list at each X-ray image capture, as an image that designates the group. Moreover, the platform table 21 can be displaced forward, backward, left, and right with the hand lever 23 and can change height with the foot pedal 24. The position information for the platform table 21 at such times is included and stored in the memory 33 as position information for the X-ray diagnosis apparatus displacer 53 of the X-ray diagnosis apparatus 1. In addition, a position directly below an exterior acromial apex on both sides of the test subject 25 while on the platform table 21, which is marked horizontally and vertically with calibration marks at every 1 cm, for example, is stored as a positional relationship between the test subject 25 and the platform table 21. When a positional offset arises between vascular contrast recorded imaging and fluoroscope imaging for the test subject 25 while on the platform table 21, the test subject 25 is moved so as to be in the same position, or the amount of positional offset is taken as a correction value and the breadth to which the platform table 21 can move horizontally and vertically is corrected. This is adopted as the new position information for the X-ray diagnosis apparatus displacer 53.

The A/D converter 31 is connected to the X-ray detector 14. The left/right-differentiated two-dimensional X-ray image data for the left eye and the right eye, which is output from the X-ray detector 14, is digitized and transmitted to the three-dimensional image composer 32 by the A/D converter 31. Figure 4 illustrates a process up to composing a three-dimensional image from the left/right-differentiated two-dimensional X-ray image data output from the X-ray detector 14 and displaying a three-dimensional image on the 3D display 44. In the three-dimensional image composer 32, of the transmitted two-dimensional X-ray image data, the left/right-differentiated two-dimensional image data for a portion necessary for three-dimensional image display is sent to the D/A converter 43, then is displayed as a three-dimensional image on the screen of the 3D display 44. Simultaneously, as shown in Figure 4, digital electrocadiogram data (hereafter, electrocadiogram data) is grouped at each image capture with position information for the X-ray diagnosis apparatus displacer 53 of the X-ray diagnosis apparatus 1, then is stored in the memory 33 (as one block) with the representative still image for the group. A user such as the doctor can set in advance, for example, that a timephase image of the electrocadiogram R-wave for three heartbeats beginning with the injection of the contrast medium is automatically selected as the representative still image for the group. Herein, that image is used as an image for the list display.

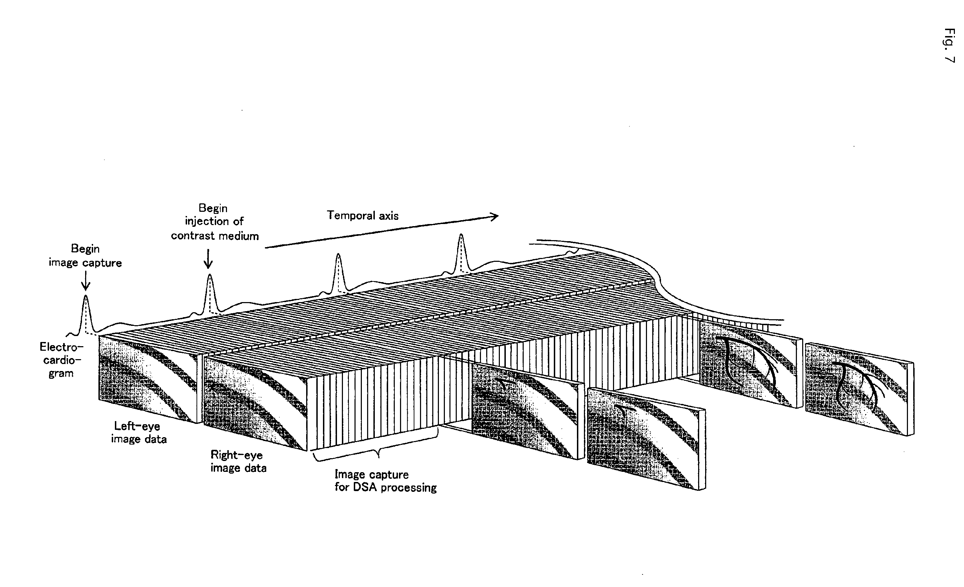

In order to obtain favorable digital subtraction (DSA) processed moving image data, as shown in Figure 7, X-ray image capture is begun matched to an appearance of the electrocadiogram R-wave during the medical procedure and injection of the contrast medium is begun matched to an appearance of the second R-wave. Thereby, a favorable vascular contrast image can be obtained around the fourth R-wave and DSA processing can be performed with the moving image for one heartbeat from the first R-wave as a DSA processing background image. The doctor can manually match the timing for the start of image capture and the start of contrast while listening to an electrocardiogram R-wave signal sound. In addition, a configuration is also possible in which, after pushing an X-ray image capture start button, X-ray image capture is begun simultaneously with the appearance of the electrocardiogram R-wave due to a command from the system controller 38 and the contrast medium is injected from an electric contrast medium injector due to a command from the system controller 38 simultaneously with the appearance of the second electrocardiogram R-wave.

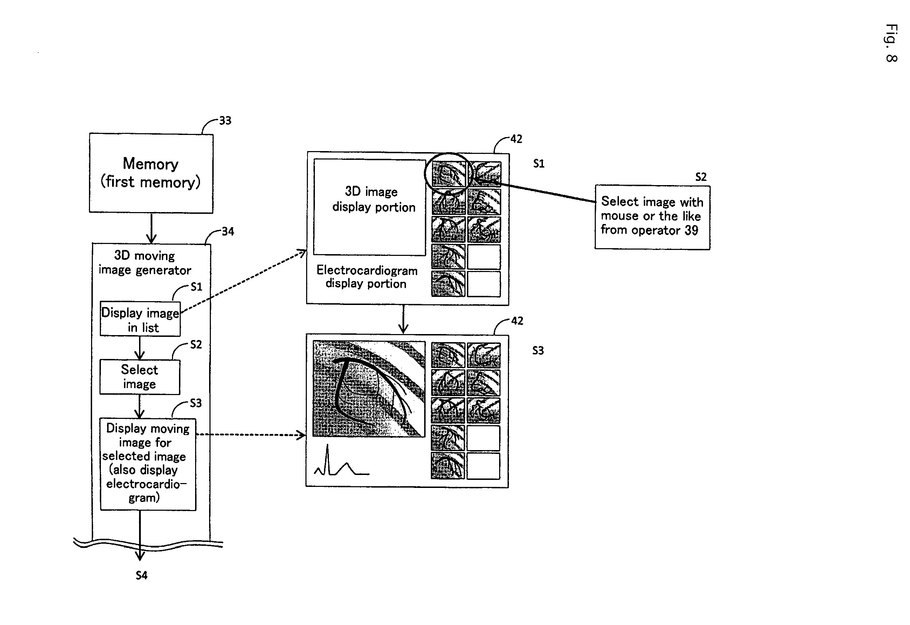

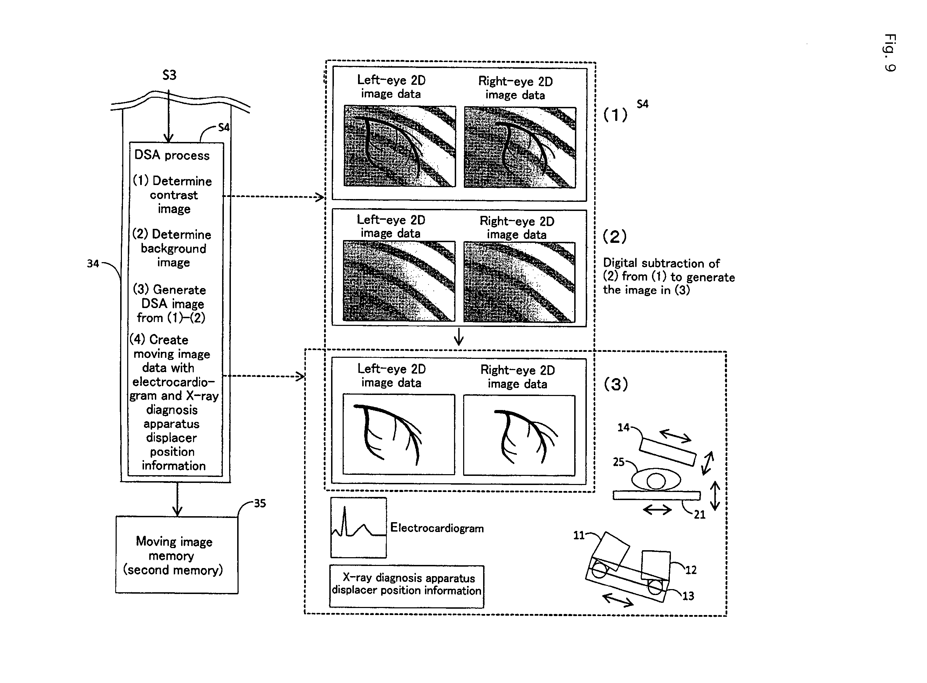

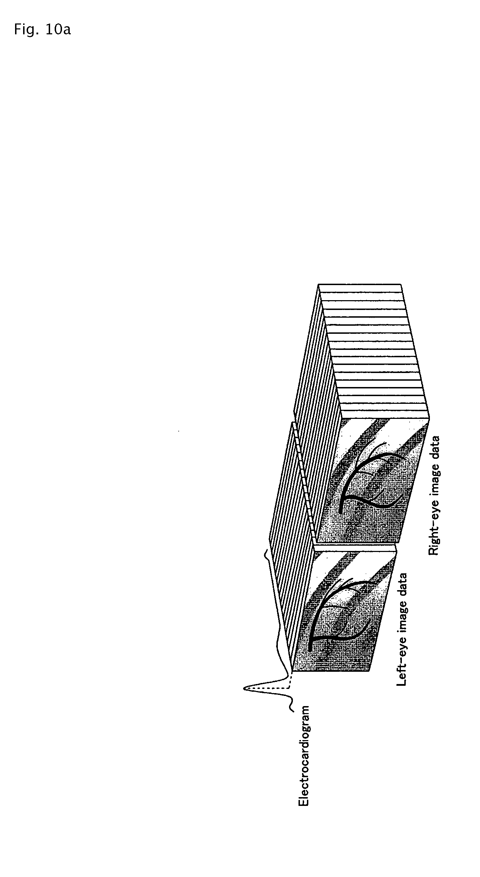

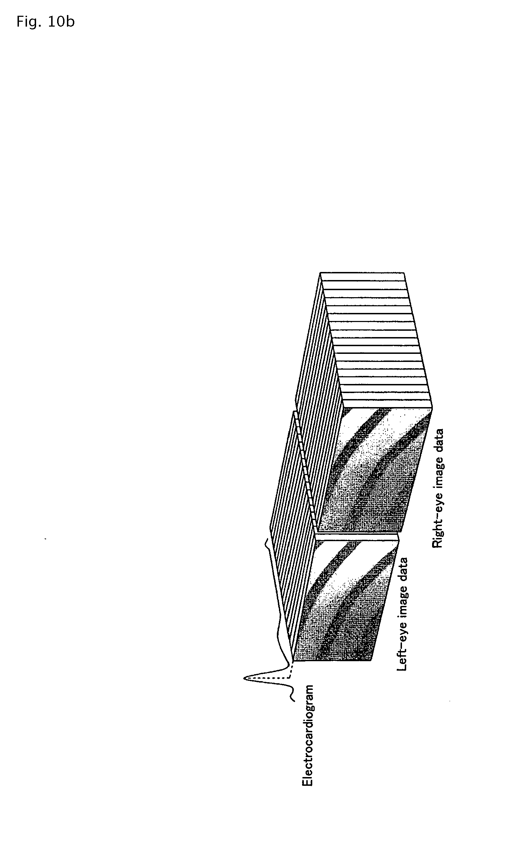

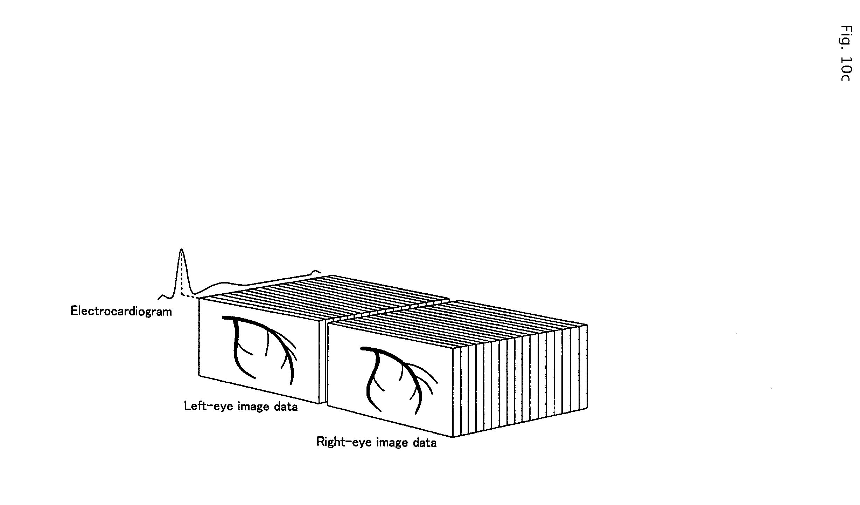

Figures 8 and 9 illustrate a process where, based on image data from the memory (first memory) 33, three-dimensional moving image data which has been DSA image processed for one heartbeat is composed and stored in the moving image memory (second memory) 35. In the three-dimensional moving image composer 34, image data stored in the memory 33 is first displayed as a list in a list display of the generator/selector display 42 (S1). Next, when the doctor operates an operator 39 such as a mouse to select one image from among these (S2), the selected moving image data is displayed in replay as a 3D moving image along with the electrocardiogram on the 3D image display portion of the generator/selector display 42 (S3). A 2D image display portion may be substituted for the 3D image display portion. In such a case, the 2D image for the left eye or the 2D image for the right eye is displayed in the 2D image display portion. While replaying the three-dimensional moving image replayed with the electrocardiogram, the doctor selects a start point and an end point in the moving image for one heartbeat with the best contrast and in the moving image for one heartbeat where no contrast medium was injected with an operation by a mouse or the like from the operator 39. As shown in Figs. 10, subtraction (DSA) processing is performed between the image data for one heartbeat with the best contrast (Fig. 10a) and the image data for one heartbeat where no contrast medium was injected (Fig. 10b) and the DSA processed image data for one heartbeat (Fig. 10c) is generated (S4). The DSA-processed three-dimensional image is stored in the moving image memory (second memory) 35 as data in which the electrocardiogram data during image capture for one heartbeat and the position information data of the X-ray diagnosis apparatus displacer 53 are associated. This string of DSA-processed three-dimensional moving images is performed for each recorded image in which the image capture angle is changed to capture an X-ray image, and is then stored in the moving image memory 35.

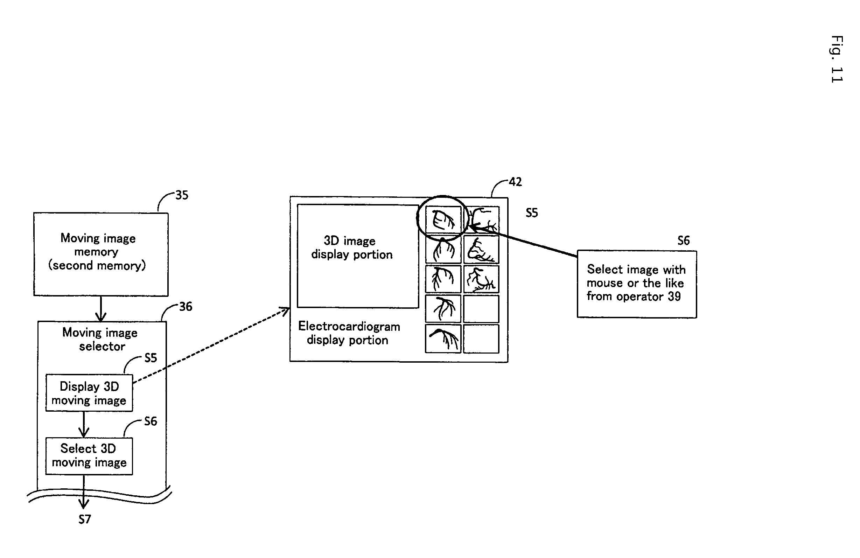

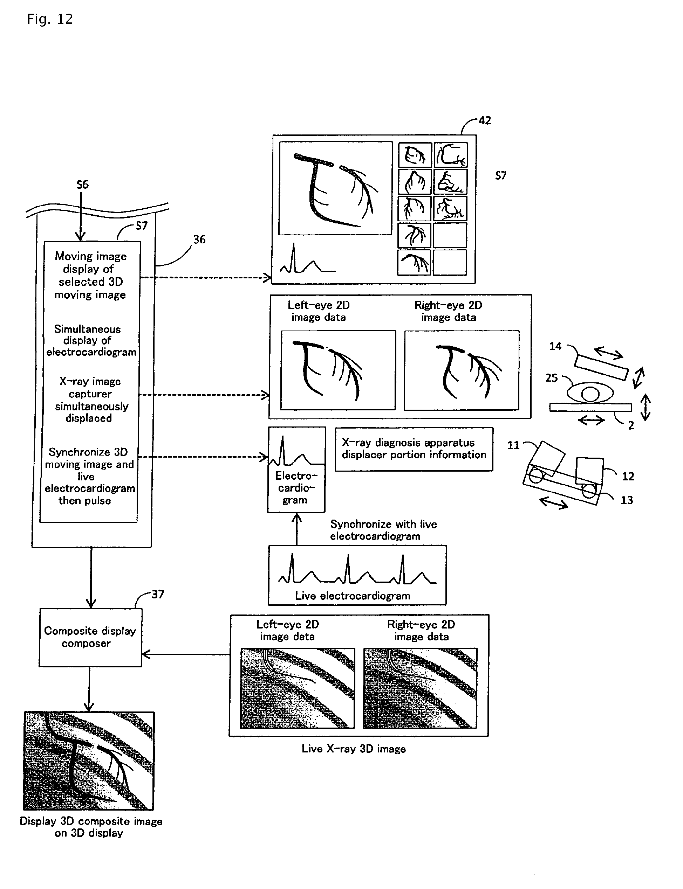

As shown in Figures 11 and 12, in the moving image selector 36, the three-dimensional vascular moving image data for one heartbeat for each image capture angle stored in the moving image memory 35 is displayed as a list in the list display portion of the generator/selector display 42 (S5). When displayed as a list, the images may be a single-frame still image for the left eye or for the right eye, rather than a three-dimensional moving image for one heartbeat. When performing a coronary artery intervention or a cardiac catheterization, the doctor selects the three-dimensional vascular moving image from the image capture angle that offers the best reference from the images from a plurality of directions displayed in the list display portion of the generator/selector display 42, with an operator 39 such as a mouse (S6). The three-dimensional vascular moving image selected with this operation is displayed in repeated replay synchronized with the live electrocardiogram. Moreover, based on the image capture angle data during capture of the three-dimensional vascular moving image from among the image capture angle data stored in the moving image memory 35, the system controller 38 automatically activates the X-ray diagnosis apparatus displacer 53 and the X-ray image capturer 10 adopts the same image capture angle as when the three-dimensional vascular moving image was captured (S7). The three-dimensional vascular moving image data is transmitted to the composite display composer 37 to be composited with the live three-dimensional X-ray images, then is transmitted to the D/A converter 43 and displayed as a three-dimensional image on the 3D display 44. Thereby, observation during three-dimensional fluoroscopy is possible at the same image capture angle as during image capture of the selected three-dimensional vascular moving image.

The system controller 38 can carry out controls in which each of the left- and right-eye images for the three-dimensional vascular moving image selected by the moving image selector 36 and the live three-dimensional X-ray image are displayed on the screen of the 3D display 44 with identical timing. Specifically, the timing for output of image data can be controlled such that the left-eye two-dimensional recorded image data (data) during projection of the X-ray beam for the left eye is sent and the right-eye two-dimensional recorded image data (data) during projection of the X-ray beam for the right eye is sent matched to the timing of the high voltage being alternatingly applied to the two X-ray tubes 11 and 12 by the high voltage generator 51. However, in the three-dimensional X-ray diagnosis apparatus, when the X-ray image capture is performed at 25 frames per second for each of the left eye and the right eye, the images are sequentially refreshed every 0.04 seconds. However, for the 0.04 seconds until the images are refreshed, the same image data continues to be output. Therefore, in the three-dimensional vascular moving image selected by the moving image selector 36, as well, the left-eye image and the right-eye image are similarly sequentially refreshed every 0.04 seconds and the same image data continues to be output as a still image for the 0.04 seconds until the image is refreshed. Therefore, even when the timing of the X-ray image capture does not match the timing of the three-dimensional vascular moving image, in which the image is refreshed every 0.04 seconds, the human eye can perceive both the live three-dimensional X-ray image and the three-dimensional vascular moving image to be displayed in composite on the 3D display 44.

As shown in Figures 11 and 12, the doctor selects one three-dimensional vascular moving image with the mouse or the like from the operator 39 from the three-dimensional vascular moving images displayed as a list in the list display portion of the generator/selector display 42. Thereby, based on the three-dimensional vascular moving image data for one heartbeat from the moving image memory (second memory) 35, the three-dimensional vascular moving image is displayed in repeated replay on the 3D image display portion of the generator/selector display 42 synchronized with the live electrocardiogram R-waves, and the image data is superimposed on the live X-ray image data and composited by the composite display composer 37. The image data composited in this way is converted to analog by the D/A converter 43 and displayed on the screen of the 3D display 44. As the 3D display 44, a commercially available three-dimensional image monitor (3D TV) can be employed. The doctor can operate the catheter or guide wire while verifying how the catheter or guide wire is proceeding through the coronary artery interior using the three-dimensional vascular moving image in the composite display.

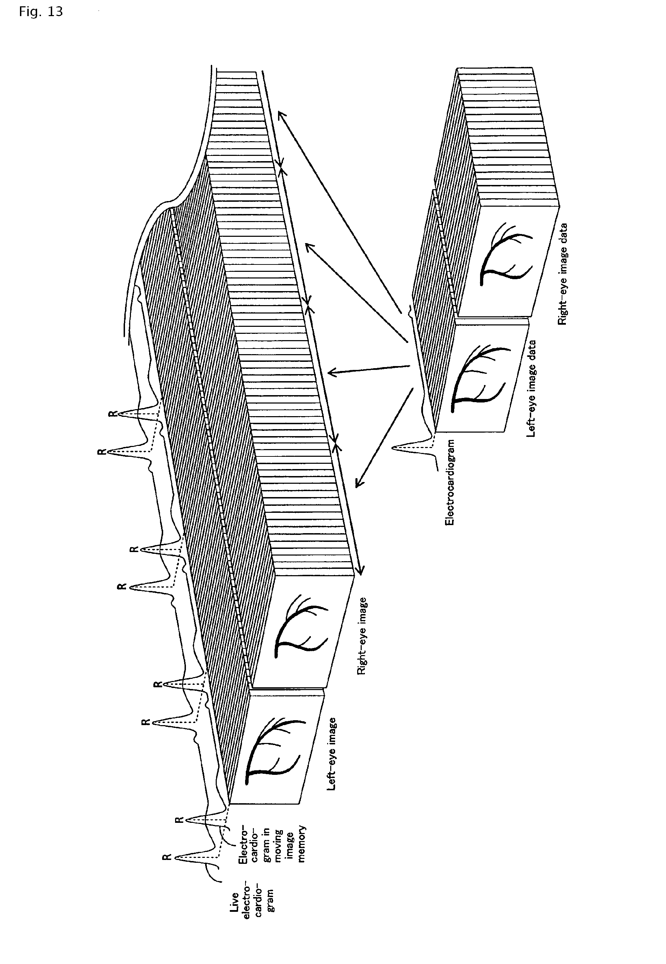

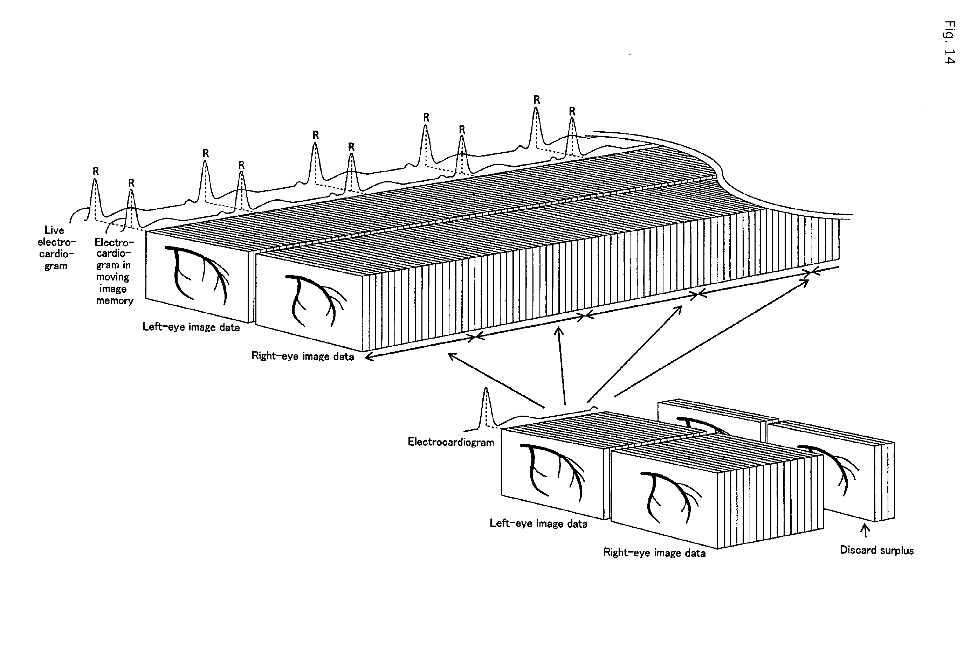

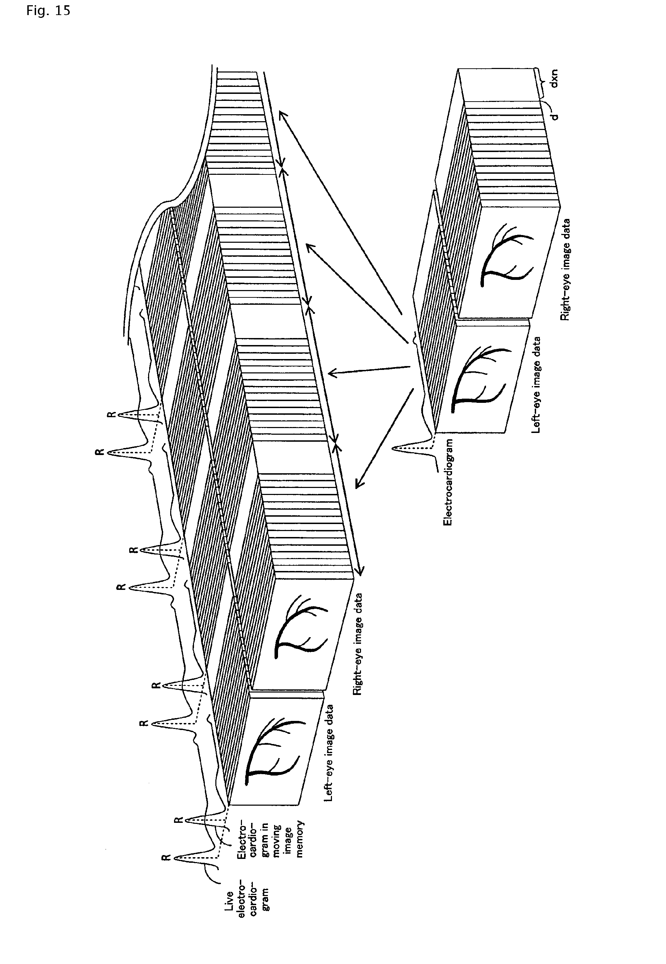

In a case where the heart rate in the live electrocardiogram substantially matches the heart rate in the recorded image, as shown in Figure 13, the electrocardiogram data for one heartbeat and the three-dimensional vascular moving image data, which are synchronized with the live electrocardiogram R-waves and stored in the moving image memory 35, may simply be repeatedly output to the composite display composer 37. In contrast, in a case where the heart rate in the live electrocardiogram is faster than the heart rate in the recorded image, as shown in Figure 14, the interval between R-waves in the electrocardiogram data for one heartbeat stored in the moving image memory 35 is shorter than the interval between R-waves in the live electrocardiograph. Therefore, data in which the surplus electrocardiograph data and the three-dimensional vascular moving image data corresponding thereto have been cut out is repeatedly output to the composite display composer 37. In addition, in a case where the heart rate in the live electrocardiogram is slower than the heart rate in the recorded image, as shown in Figure 15, the interval between R-waves in the electrocardiogram data for one heartbeat stored in the moving image memory 35 is longer than the interval between R-waves in the live electrocardiogram. Therefore, of the three-dimensional vascular moving image data for one heartbeat, final three-dimensional vascular moving image data d continues to be output for n seconds until the next live electrocardiogram R-wave appears, and thus becomes a still image for n seconds. Moreover, in a case where the interval between R-waves in the live electrocardiogram is irregular, such as in atrial fibrillation or extrasystole, compensation is performed by combining the process for when the heart rate in the live electrocardiogram is fast and the process for when the heart rate is slow, as described above.

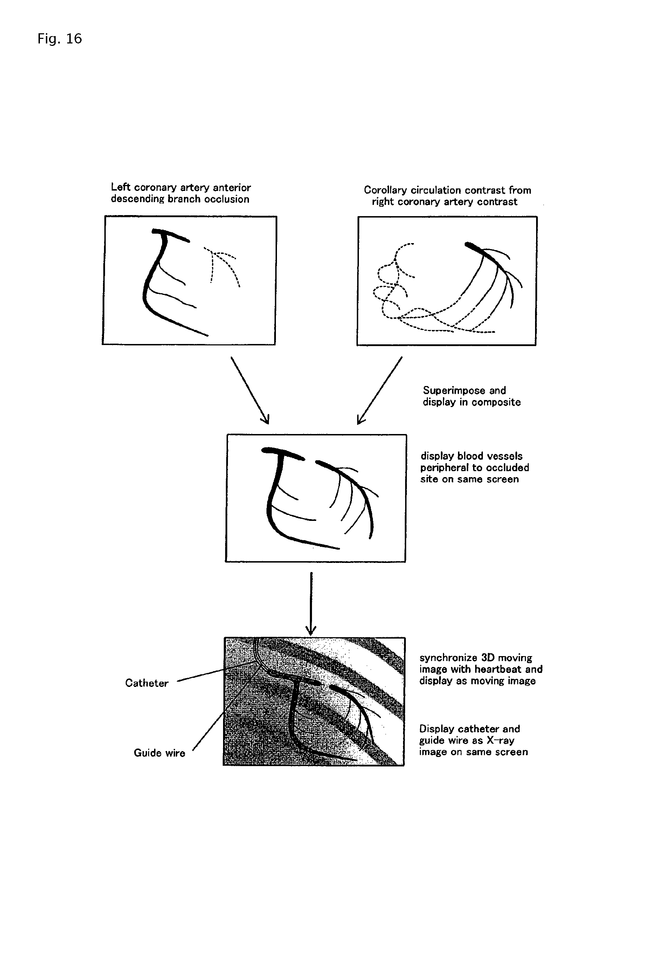

Figure 16 illustrates a compositing process for an occluded coronary artery branch, a coronary artery branch peripheral to the occluded area which has received contrast medium through collateral circulation, and X-ray images taken during the medical procedure, as well as a catheter and a guide wire. In a conventional method, when an intervention is performed on a fully occluded left anterior descending branch, the contrast medium is injected into the right coronary artery to perform contrast of the periphery of the left anterior descending branch via collateral circulation. Feeding of the guide wire through the fully occluded site and insertion to the periphery was thus confirmed. Therefore, insertion of another catheter for contrasting the right coronary artery was required.

However, when the X-ray diagnosis apparatus 1 of Example 1 is used, this problem can be resolved. Specifically, as shown in Figure 16, the occluded site of the left anterior descending branch is imaged in advance and, additionally, the right coronary artery is imaged from the same angle. The periphery of the left anterior descending branch then receives the contrast medium via collateral circulation, and images thereof are stored in the memory 33. From the two groups of image data stored in the memory 33, images in which the left anterior descending branch is occluded in the contrast image of the left coronary artery and images in which the periphery of the left anterior descending branch receives the contrast medium via collateral circulation with the right coronary artery contrast are selected as moving images for one heartbeat and are DSA processed by the DSA processor 55. The two groups of three-dimensional vascular moving image data obtained are stored in the moving image memory 35, then the two groups of three-dimensional vascular moving image data are selected by the moving image selector 36 and composited by the composite display composer 37, are further composited by the composte display composer 37 with the live three-dimensional X-ray images, and the composite image data is sent to the D/A converter 43 to be displayed as a three-dimensional image on the 3D display 44. In a case of composite display, a color-differentiated display is performed in which, for example, the three-dimensional moving image of the left coronary artery is displayed in red, the left anterior descending branch periphery which receives the contrast medium via collateral circulation from the coronary artery is displayed in yellow, and the catheter and guide wire in the live three-dimensional X-ray image are displayed in black. Thereby, visualization of each is facilitated.

In this way, the occluded left anterior descending branch and the blood vessels peripheral to the occluded site are displayed in repeated replay as three-dimensional vascular moving images synchronized with the live electrocardiogram R-waves. By being superimposed on the live three-dimensional X-ray image and differentiated with colors, a composite display is performed on the screen of the 3D display 44. Therefore, the doctor can stereoscopically visualize how the guide wire is advancing through the occluded site and how the guide wire is being fed through the occlusion to be inserted into the peripheral blood vessel. Thereby, the intervention can be performed more safely and accurately. Moreover, there is no need to employ another catheter nor to perform collateral circulation contrast. In addition, as the X-ray image capturer 10, an apparatus is employed having the single support arm 16, in which the two X-ray tubes 11 and 12 are disposed on one end in the length direction thereof and the single X-ray detector 14 is disposed at the second end in the length direction thereof; and an image capture angle changing means 53 rotating centered on the rotation shaft 18, which is orthogonal to the length direction of the support arm 16, and displacing in the length direction of the support arm 16. Therefore, three-dimensional X-ray image capture from any angle sought by the doctor becomes possible.

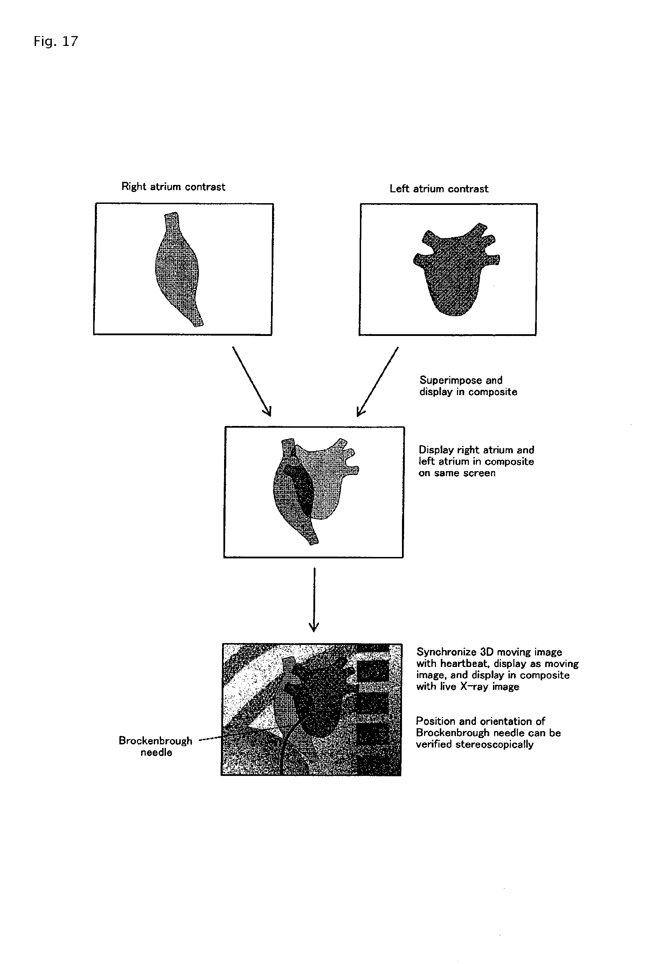

Figure 17 illustrates an image compositing process for a right atrium which receives the contrast medium by right atrial contrast, a left atrium which receives the contrast medium later, and an X-ray image taken during the medical procedure; and a Brockenbrough needle going toward an atrial septum. Recently, catheter ablation has been performed as a treatment for atrial fibrillation. In catheter ablation, the atrial septum is pierced in the Brockenbrough technique and a catheter inserted from the right atrium interior to the left atrium interior, then an electrode catheter is placed against an appropriate location of a pulmonary vein or the left atrium interior, electricity is conducted therethrough, and heart muscle is burned away. However, in this type of procedure, determination of a site for piercing the atrial septum and determination of a location to place the electrode catheter against has been difficult with methods that use X-ray images obtained from conventional X-ray diagnostic apparatuses.

In the three-dimensional X-ray diagnostic apparatus 1 of Example 1, as shown in Figure 17, the contrast medium is first injected into the right atrium and right atrial contrasting is performed. Thereby, the contrast medium flows from within the right atrium through the right ventricle, the pulmonary artery, and the pulmonary vein into the left atrium. Then, three-dimensional X-ray image capture is performed until the right atrium receives the contrast medium and the left atrium receives the contrast medium, then the electrocardiogram data is stored in the memory 33 along with the position information data for the X-ray diagnosis apparatus displacer 53. The contrast image of the right atrium and the contrast image of the left atrium are selected by the three-dimensional moving image generator 34 from the X-ray image data in the memory 33. The moving images of the right atrium and the left atrium for one heartbeat in the selected image data are generated by DSA processing and are then stored in the moving image memory 35 with the electrocardiogram data and the position information data for the X-ray diagnosis apparatus displacer 53. The moving images for one heartbeat stored in the moving image memory 35 are displayed in a list on the generator/selector display 42, then the doctor selects the moving images of the right atrium and the left atrium from among them. The moving images are superimposed in different colors from one another and composited by the composite display composer 37. An image signal in which the moving images for one heartbeat are synchronized with the live electrocardiogram R-wave and then looped is transmitted to the D/A converter 41 and displayed in replay on the 3D image display portion of the generator/selector display 42. The image data in which the moving images are further composited by being superimposed on the live X-ray image is transmitted to the D/A converter 43 and displayed on the 3D display 44.

In the 3D display 44, the right atrium is displayed in blue, the left atrium in red, and the live X-ray image of the catheter and the guide wire in black, for example, each in a different color so as to facilitate visualization of each. The doctor is able to stereoscopically visualize the location of the atrial septum and the orientation of the forefront of the Brockenbrough needle, which is bent in a "J" shape, and therefore the atrial septum can be pierced safely and accurately. Moreover, even when performing an operation to press the forefront of the electrode catheter for ablation against an appropriate site in the left atrium interior or the vicinity of the pulmonary vein, the operation can be performed accurately in a short amount of time.

However, in a case where identification of the location of the atrial septum is difficult due to being a post-operative heart or due to deformity, when piercing the atrial septum with the Brockenbrough technique, there are cases where the location of the atrial septum will not be ascertained simply by superimposing the image of the right atrium and the image of the left atrium. In such a case, as shown in Figures 1 and 2, based on three-dimensional exterior image data for stereoscopic images or cross-sections of the focus area taken from a variety of angles obtained from another imaging diagnosis apparatus (the X-ray computed tomography apparatus 61, the cardiac ultrasound apparatus 62, the magnetic resonance imaging apparatus 63) that was stored in the DICOM (Digital Imaging and Communication in Medicine) image server 60, with an operation on the mouse and the like from the operator 39, the doctor selects and loads a needed three-dimensional image via the interface 65 through a network line, then stores the image in the exterior image memory 66. The three-dimensional image data generates two-dimensional image data in which a three-dimensional image is viewed from two directions for the left eye and the right eye, for example by applying a 20° angle, through a computer digital image process with the three-dimensional image composer 67, and is then stereoscopically displayed on a screen of the exterior image display 46 (Figures 1 to 3). In the three-dimensional exterior image composer 66, a three-dimensional exterior image of the focus area on the test subject displayed as an image on the image display 46 is digital image processed by the computer. Then, by operating the mouse of the operator 39 while viewing the image, the doctor can resize the image to a desired size, can rotate the three-dimensional image to view from a desired angle, can view a cross-section cut on a desired slice face of the three-dimensional image, and so on, while observing in comparison with the composite display screen of the vascular moving image of the focus area and the X-ray image taken during the medical procedure displayed stereoscopically on the 3D display 44.

As necessary, the composite display composer 37 superimposes the three-dimensional exterior image on the X-ray image taken during the medical procedure or on the moving image of the left atrium and the right atrium and displays these in composite on the 3D display 44. Thereby, determination of the location of the atrial septum can be performed more accurately. Moreover, a cardiac ultrasound image taken during the medical procedure is obtained from the internal ultrasound imaging apparatus 64 by inserting an ultrasound catheter into the right atrium and is displayed as an image on the exterior image display 46 via the interface 65 through the network line. The site where the ultrasound catheter is thought to be touching the atrial septum can thus be confirmed with the ultrasound image taken during the medical procedure. At this time, the orientation of the ultrasound catheter, which is bent in a "J" shape, can be confirmed on the 3D display 44 with the three-dimensional X-ray image taken during the medical procedure. Thus, confirmation of the location of a safe piercing site in the atrial septum can be performed more accurately. Moreover, the three-dimensional X-ray image of the ultrasound catheter is stored in the memory 33 and the three-dimensional moving image for one heartbeat is generated by the three-dimensional moving image generator 34, then is stored in the moving image memory 35. Next, the 3D display 44, on which the composite images of the right atrium and the left atrium are displayed in composite in different colors, further displays the three-dimensional image of the ultrasound catheter in composite in a different color, then displays these images in composite with the three-dimensional X-ray image taken during the medical procedure. Thereby, the most appropriate piercing site can be confirmed during the Brockenbrough technique. Moreover, when X-ray images are continuously viewed on the screen of the 3D display 44 during the medical procedure, eye fatigue is likely to occur. Therefore, switching between 2D and 3D can also be enabled so that, when performing a simple procedure, an image for only the left eye, for example, can be displayed as a two-dimensional image and a three-dimensional image is displayed only when the focus area is reached.

Moreover, by using the 3D display 44, which is a color display, image compositing can be performed using the properties of the three elementary colors of light. Therefore, even when DSA processing is not performed, the shadow images of bones and soft organs in the composite image do not become dark. Specifically, in the conventional method employing a display with a black-and-white screen, there is a risk that portions where the shadow images of bones and soft organs overlap in the recorded images and the live X-ray images will become twice as dark and that in the recorded vascular moving images and X-ray images, the shadows of the catheter and the guide wire will become difficult to see. In contrast, herein, color screens are used for all of the composite image display apparatuses 40. Thus, on the screens of the generator/selector display 42 and the 3D display 44, when the recorded images are colored red, for example, and the live X-ray images are colored blue, for example, by the composite display composer 37, the bones and soft organs where the recorded images and the live X-ray images overlap become reddish-purple (magenta), the vascular images recorded with vascular contrast are displayed in red, and the catheter and guide wire in the live X-ray images are displayed in blue. Moreover, because these images are displayed on the 3D display 44, differences in distance (depth) are also present and the shadows of bones and soft organs do not interfere when viewing the shadows of the vascular images, catheter, and guide wire.

In addition, when the catheter and guide wire are inserted into the blood vessels, superimposed on the vascular image, the guide wire in the vascular shadows (displayed in red) is displayed in reddish-purple. Moreover, there are various color combinations for the background color, which is not colored in the recorded images and the live X-ray images, such as remaining black or coloring a dark green, and these color combinations can be selected as desired by a mouse operation of the operator 39. Further, in a case where two images having different temporal axes are superimposed for composite display, of the contrast images, for example, a left coronary artery image, when colored red, is displayed in red up to an occluded coronary artery branch; a periphery of the left coronary artery, which has received contrast from the right coronary artery via collateral circulation, is colored blue; and the live X-ray images of the catheter and guide wire are displayed in dark green. In such a case, the shadows of the bones and soft organs are displayed in light gray. Accordingly, the view is such that the guide wire (dark yellow) advances through the left coronary artery (red) and the guide wire (dark cyan) is fed through the occluded site in the blood vessel and proceeds into the peripheral blood vessel (blue).

In this way, by employing color screens in the image display apparatus 40 to display the recorded images and X-ray images each in different colors with the composite display composer 37, there is no further need for DSA processing in the three-dimensional moving mage generator 34. As a result, capturing a background image for DSA processing becomes unnecessary. Therefore, in image processing by the three-dimensional moving image generator 34 of Figs. 8 and 9, an operation in the DSA processing S4 selects one heartbeat with the best contrast (1), then converts that image data to data associated with the electrocardiogram data and the position information data for the displacer of the X-ray image capture apparatus from when the image data was captured (4), which is then stored in the moving image memory (second memory) 35. Thus, in the image processing by the three-dimensional moving image generator 34, there is no need to perform operations to obtain a background image in the DSA processing S4 (2) nor to erase the background image from the contrast image based thereon (3), and so the operation is simplified.

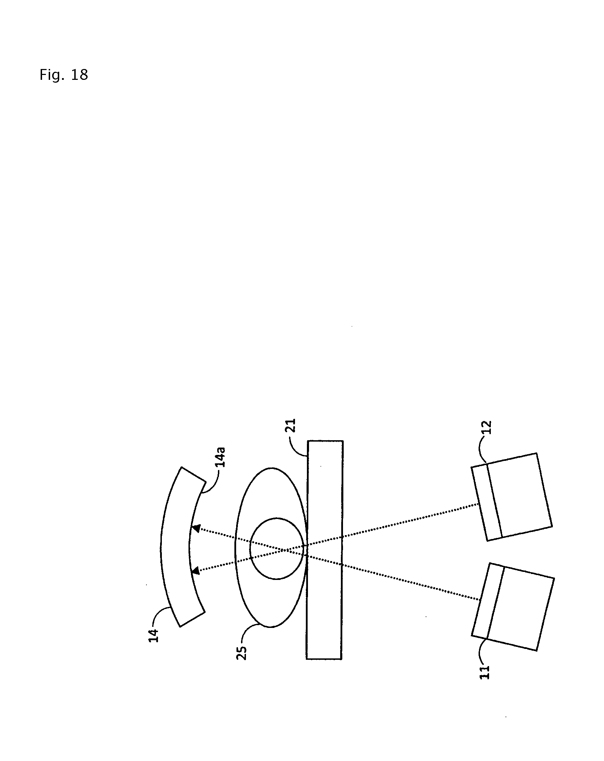

In the case of Example 1, one X-ray detector 14 is used. Thus, when the X-ray detector surface 14a is flat, the X-ray beams from the two X-ray tubes 11 and 12 are projected at an angle. However, as shown in Fig. 18, an apparatus having a concave X-ray detector surface 14a is used as the X-ray detector 14 in the X-ray image capturer 10. Thus, the X-ray beams projected from the two X-ray tubes 11 and 12 alternatingly reach the concave X-ray detector surface 14 of the single X-ray detector 14 at an angle nearly orthogonal thereto after intersecting at the focus area on the test subject 25. As a result, the degree of distortion in the X-ray images of the test subject 25 detected by the X-ray detector 14 can be reduced as compared to a case where the X-ray detector surface 14a is a flat surface. Thereby, as in the case of Example 1, there is no need to correct distortion due to digital image processing in the three-dimensional image composer 32.

### Example 2

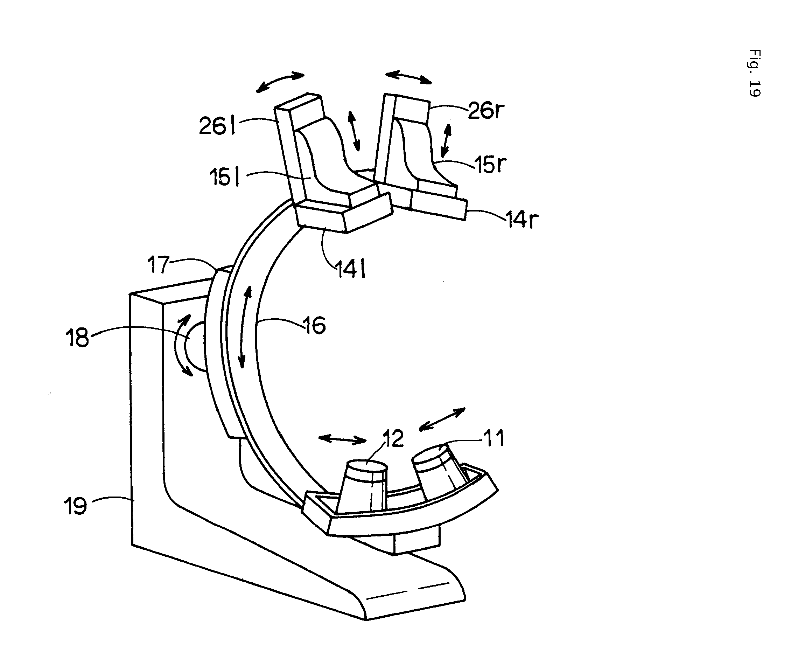

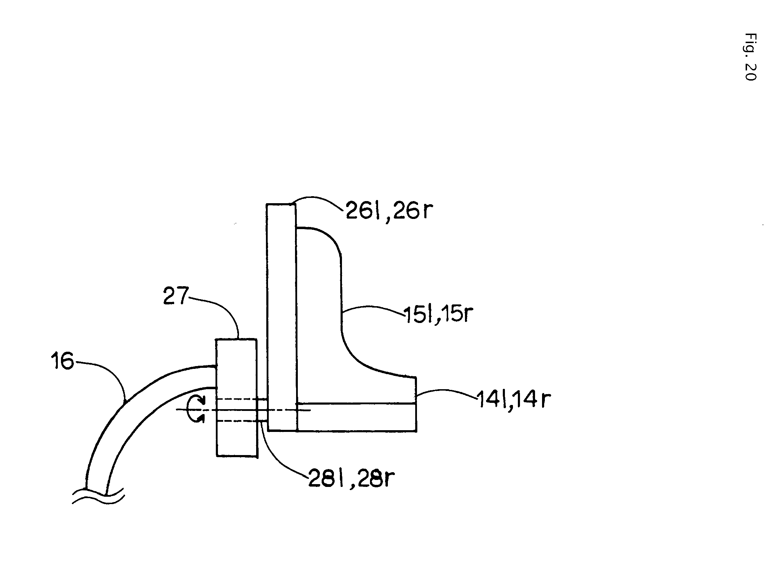

Next, an X-ray diagnosis apparatus 1 according to Example 2 of the present invention is described with reference to Figures 19 and 20. The particular feature of the X-ray diagnosis apparatus 1 according to Example 2 of the present invention is that two X-ray detectors 141 and 14r are employed as the X-ray detector 14 of the X-ray image capturer 10. Thereby, the two X-ray detectors 141 and 14r and the two X-ray tubes 11 and 12 are disposed in a one-to-one relationship facing each other on one support arm 16. As a result, the X-ray beams projected from one of the X-ray tubes 11 and 12 is detected by one of the X-ray detectors 141 and 14r, and the X-ray beam projected from the other of the X-ray tubes 11 and 12 is detected by the other of the X-ray detectors 141 and 14r. Accordingly, image distortion and displacement of the center in the X-ray images are unlikely to occur. Moreover, for the two X-ray tubes 11 and 12, a high voltage from the high voltage generator 51 can be applied to both simultaneously.

The two X-ray detectors 141 and 14r are provided to one support arm 16 so as to be displaceable in a projection direction of both X-ray beams (a separation direction of the X-ray detectors 141 and 14r from the X-ray tubes 11 and 12) and so as to be displaceable in a direction orthogonal to the projection direction of both X-ray beams. Specifically, a support arm attachment base 27 is fixed to the second end portion of the support arm 16 and base portions of a pair of rotation shafts 281 and 28r are fixed to a lower portion of the support arm attachment base 27. On a front portion of both rotation shafts 281 and 28r is fixed a lower end portion of a pair of rotation base boards 261 and 26r which rotate each of the two X-ray detectors 141 and 14r in a direction orthogonal to the projection direction of both X-ray beams. Both of the X-ray detectors 141 and 14r are fixed to a bottom end portion of a pair of drivers 151 and 15r, which are disposed on a front surface of the pair of rotation base boards 261 and 26r. In such a case, the rotation shafts 281 and 28r are stored in the memory 33 as the position information for the X-ray diagnosis apparatus displacer 53.

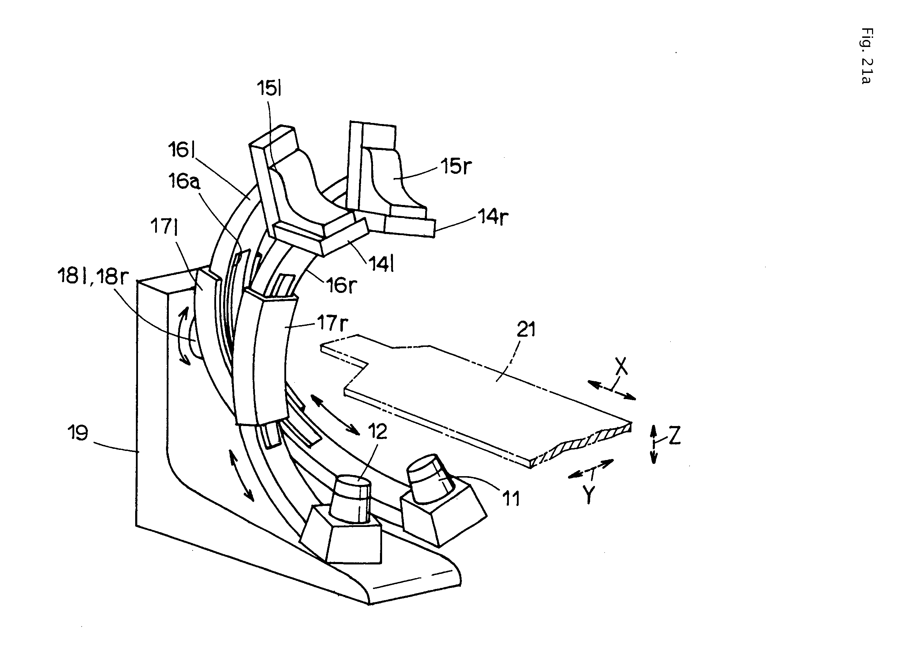

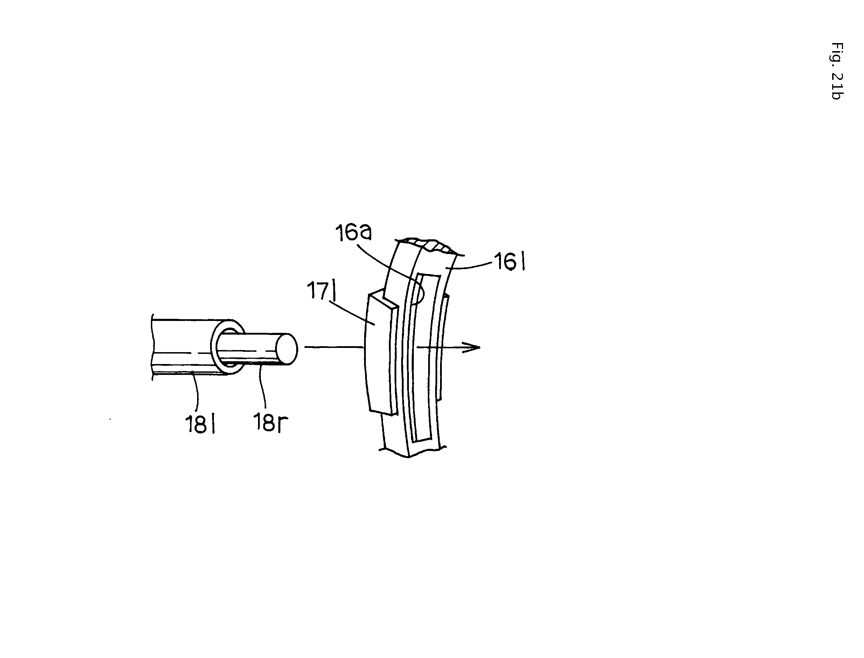

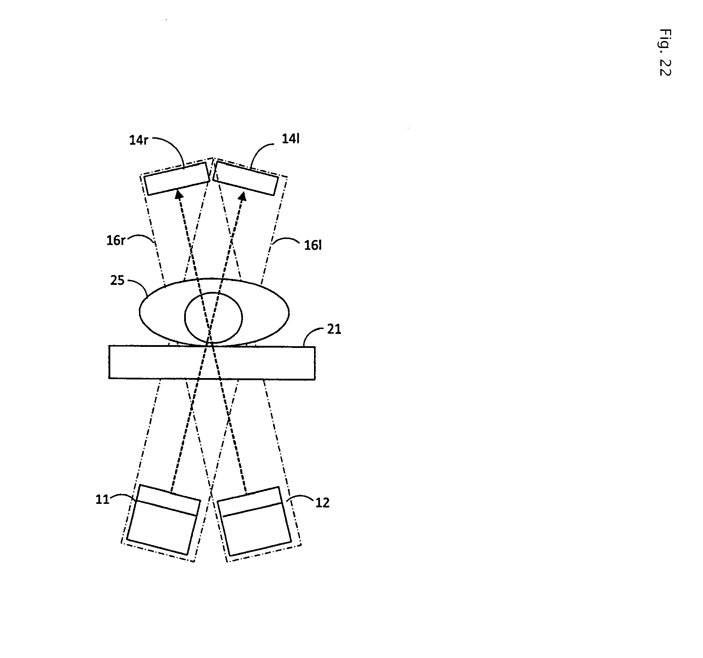

Next, the X-ray diagnosis apparatus 1 according a different Example 2 of the present invention is described with reference to Figures 21 and 22. The particular feature of the X-ray diagnosis apparatus 1 according to the different Example 2 of the present invention is that two substantially "C"-shaped support arms 161 and 16r are provided in a crossed state to the X-ray image capturer 10, one each of the X-ray tubes 11 and 12 is provided to one end portion of both of the support arms 161 and 16r, and one each of the X-ray detectors 141 and 14r is provided to a second end portion of both of the support arms 161 and 16r. In such a case, both of the support arms 161 and 16r are attached in a state crossing each other to the rotation shafts 181 and 18r of a support stand 19 via two rotators 171 and 17r. Moreover, the rotation shaft 181 is a circular tubular body (sheathing shaft) and the rotation shaft 18r is a circular columnar body (core shaft) inserted coaxially into an interior space of the rotation shaft 181. Thus, when the image capture angle changes, the two support arms 161 and 16r can be simultaneously rotated in the same direction to the same angle and can also be simultaneously displaced the same distance to the same side of the length direction by the X-ray diagnosis apparatus displacer 53. As a result, the X-ray beams can be projected head-on onto the X-ray detector surfaces 14a of the X-ray detectors 141 and 14r. Therefore, X-ray projection can be performed simultaneously from the two X-ray tubes 11 and 12 without distortion developing in the X-ray image and without producing displacement in the centers of the X-ray images.

In other words, the X-ray diagnosis apparatus 1 according to the different Example 2 of the present invention provides various differentiated driving systems and control systems. Two "C"-shaped support arms 161 and 16r are provided overlapping on top of each other in a crossed state and rotatable around a side surface of the platform table 21 centered on the rotation shafts 181 and 18r, which extend horizontally in a core-in-sheath shape from the support stand 19 toward the platform table 21, the support stand 19 being positioned further outward than one end in the length direction of the platform table 21. One each of the X-ray tubes 11 and 12 is provided to one end portion of each of the support arms 161 and 16r. In addition, one each of the X-ray detectors 141 and 14r is provided to the second end portion of each of the support arms 161 and 16r, the X-ray detectors 141 and 14r receiving the X-ray beams projected from the corresponding X-ray tube 11 and 12. An elongated aperture 16a extending in the length direction is formed at a central portion in the length direction on both of the support arms 161 and 16r. A shaft aperture is formed on a back plate of a curved slide guide having a trench shape and configuring the main body of the rotator 171 for the support arm 161, which is positioned to the rear of the elongated aperture 16a. Positioned to the front thereof, a slide guide curved in a circular arc shape and configuring the main body of the rotator 17r for the support arm 16r has a square cylindrical shape. Further included are the circular tubular rotation shaft 181 and the circular columnar rotation shaft 18r, which is inserted into the interior space of the rotation shaft 181 and the foremost end of which projects from the opening on the forefront of the rotation shaft 181. Of these, the foremost end of the rotation shaft 181 is fixed to a portion on the back plate of the slide guide of the rotator 171 where the shaft aperture is formed and the foremost end of the rotation shaft 18r passes through the elongated aperture 16a of the support arm 161, which is positioned to the rear, and is fixed to the back plate of the slide guide of the rotator 17r for the support arm 16r, which is positioned to the front. Moreover, the X-ray diagnosis apparatus displacer (image capture angle changing means) 53 rotates each of the rotation shafts 181 and 18r and simultaneously rotates the two support arms 161 and 16r in the same direction to the same angle. In addition, the two support arms 161 and 16r are simultaneously displaced to the same side in the length direction by the same distance via both of the rotators 171 and 17r.

With such a configuration, the two support arms 161 and 16r can be simultaneously rotated in the same direction to the same angle by the X-ray diagnosis apparatus displacer (image capture angle changing means) 53 via the rotation shafts 181 and 18r. In addition, the two support arms 161 and 16r can be simultaneously displaced to the same side in the length direction by the same distance via both of the drivers 171 and 17r, and the X-ray beams being projected from the two X-ray tubes 11 and 12 can be projected toward each of the X-ray detectors 141 and 14r directly opposite.

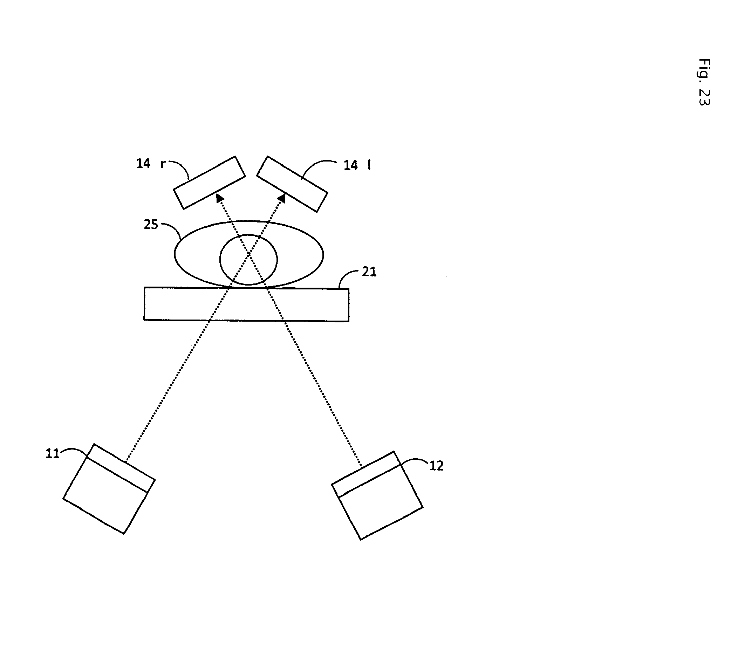

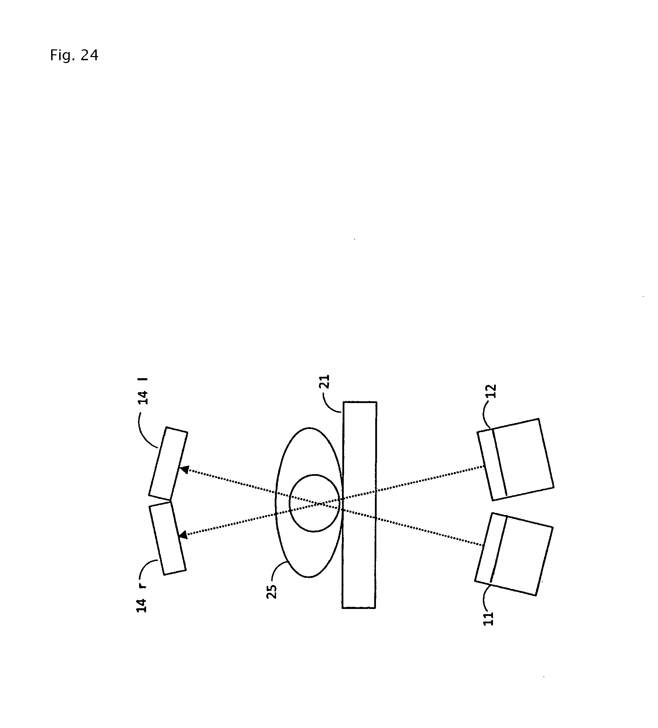

During X-ray image capture with Example 2, which employs the two X-ray detectors 141 and 14r, in order to obtain favorable X-ray images, the closer the X-ray detectors 141 and 14r are brought to the test subject 25, the larger the angle of intersection for the two X-ray beams will be at the test subject 25, as shown in Figure 23. When the doctor observes the three-dimensional image rising from the screen of the 3D display 44, the impression of the 3D image leaping out of the screen will become too strong and either the doctor will only be able to view the image for a short time due to eye fatigue or will no longer be able to view the image as 3D. In this regard, for a portion of low importance in the focus area on the test subject 25, fluoroscopy is performed using only one set, for example the X-ray tube 11 and the X-ray detector 141, and the recorded images used are also put into a composite display with two-dimensional moving images to perform the coronary artery intervention. In addition, the two sets of the X-ray tubes 11 and 12 and the X-ray detectors 141 and 14r may also be used only at a site where operation of the guide wire is difficult, such as in a vascular branch. However, operation becomes troublesome. In order to remove this burden, the X-ray detectors 141 and 14r may be moved away from the test subject 25, as shown in Figure 24 (also see Figure 22), or fluoroscopy and X-ray image capture may be performed in a state where the angle of intersection of the two X-ray beams has already been narrowed. The quality of the X-ray image is thereby reduced; however, the effect of the 3D image leaping out of the screen becomes easier to view. Examples of the present invention have been described above; however, the present invention is not limited to the above-described embodiments and design modifications may be made within the scope of the description of the invention.

### Industrial Applicability

In the present invention, X-ray images taken during a medical procedure can be viewed in three-dimensional images which appear to rise out of a screen on a 3D display, and thus offer important image information during performance of a procedure during a coronary artery intervention and a cardiac catheterization. As a result, the present invention is useful as an X-ray diagnosis apparatus that highly contributes to the safety and increased procedural efficiency of an intervention and catheterization.

### Description of Reference Numerals

| | |
|---|---|
| 1 | :X-ray diagnosis apparatus, |
| 10 | :X-ray image capturer, |
| 11, 12 | :X-ray tube, |
| 14, 14l, 14r | :X-ray detector, |
| 14a | :X-ray detector surface, |
| 16, 16l, 16r | :Support arm, |
| 18, 18l, 18r | :Rotation shaft, |
| 21 | :Platform table, |
| 25 | :Test subject, |
| 32 | :Three-dimensional image composer, |
| 33 | :Memory (first memory), |
| 35 | :Moving image memory (second memory), |
| 37 | :Composite display composer (three-dimensional image composer), |
| 38 | :System controller, |
| 44 | :3D display (color display), |
| 51 | :High voltage generator, |
| 53 | :X-ray diagnosis apparatus displacer (image capture angle changing means) |
| 54 | :Image capture angle detecting means. |

## Claims

1. An X-ray diagnosis apparatus comprising:
an X-ray image capturer capturing three-dimensional X-ray images of a focus area on a test subject, and
a three-dimensional image processor obtaining three-dimensional moving images of the focus area which are used during a catheterization of the focus area based on X-ray image data captured by the X-ray image capturer, wherein
the X-ray image capturer comprises:
two X-ray tubes projecting X-ray beams from two different directions toward the test subject atop a platform table;
one or two X-ray detector(s) obtaining X-ray image data of the test subject from the two different directions by detecting the X-ray beams projected from the two X-ray tubes;
an image capture angle changing means changing an image capture angle of the test subject when X-ray images of the focus area on the test subject are captured using the two X-ray tubes and the one or two X-ray detector(s); and
an image capture angle detecting means detecting the image capture angle of the test subject when X-ray images of the focus area on the test subject are captured using the two X-ray tubes and the one or two X-ray detector(s), and
the three-dimensional image processor comprises:
a three-dimensional image composer composing three-dimensional image data by correcting, with digital image processing, center-offset in an area of interest in X-ray image data of the test subject from the two different directions that was detected by the one or two X-ray detector(s) and distortion of the X-ray image data of the test subject from the two different directions;
a memory in which the three-dimensional image data composed by the three-dimensional image composer and vascular image data of the focus area where a contrast medium has been injected are each associated and stored with electrocardiogram data and image capture angle data from the image capture angle detecting means taken during X-ray image capture of the test subject;
a moving image memory storing three-dimensional vascular moving image data for at least one heartbeat that was generated by associating each of the electrocardiogram data and the image capture angle data from the image capture angle detecting means taken during X-ray image capture of the test subject with the vascular image data for at least one heartbeat from the vascular image data of the focus area stored in the memory;
a system controller changing the image capture angle of the test subject with the image capture angle changing means such that the image capture angle is the same as the image capture angle data associated with the three-dimensional vascular moving image data stored in the moving image memory;
a 3D display stereoscopically displaying a three-dimensional vascular moving image for at least one heartbeat that was generated based on the three-dimensional vascular moving image data for at least one heartbeat stored in the moving image memory and a three-dimensional X-ray image obtained by capturing an X-ray image of the focus area during the medical procedure with the X-ray image capturer from the two different directions, such that each stands out from a screen; and
a three-dimensional image compositer in which the three-dimensional vascular moving image for at least one heartbeat (stored in the moving image memory) is synchronized to an electrocardiogram of the test subject during the medical procedure and is repeatedly replayed on a screen of the 3D display and, after changing the image capture angle of the test subject with the system controller, the three-dimensional vascular moving image for at least one heartbeat that is being repeatedly replayed and the three-dimensional X-ray images of the focus area taken during the medical procedure are substantially synchronized and stereoscopically displayed superimposed on the screen of the 3D display.

2. The X-ray diagnosis apparatus according to claim 1, wherein:
the X-ray image capturer comprises:
a support arm on which two X-ray tubes are disposed at one end in a length direction and one X-ray detector is provided at a second end in the length direction facing the two X-ray tubes; and
a high-voltage generator supplying power alternatingly to the two X-ray tubes such that the X-ray beams from the two X-ray tubes are not simultaneously projected onto the single X-ray detector, and
the image capture angle changing means rotates the support arm around a rotation shaft orthogonal to the length direction of the support arm and displaces the support arm in the length direction of the support arm.

3. The X-ray diagnosis apparatus according to claim 1 or claim 2, wherein there is one X-ray detector and the X-ray detection surface of the X-ray detector has a concave surface.

4. The X-ray diagnosis apparatus according to claim 1, comprising a three-dimensional exterior image composer generating two-dimensional image data from the two directions necessary in order to stereoscopically display on an exterior image display a three-dimensional exterior image of the focus area, which was generated based on three-dimensional exterior image data of the focus area on the test subject obtained from a second imaging diagnosis apparatus; and performing at least one of changing an image size of the three-dimensional exterior image, changing a viewing angle of the three-dimensional exterior image, viewing a cross-section at a predetermined position in the three-dimensional exterior image, and composite display of the three-dimensional exterior image on the 3D display screen superimposed on the three-dimensional vascular moving image stored in the moving image memory and the three-dimensional X-ray image taken during the medical procedure, and wherein
an intracardiac ultrasound image captured during the medical procedure by an internal ultrasound imaging apparatus using an ultrasound catheter is viewed in comparison with the three-dimensional X-ray image taken during the medical procedure, is replayed as a moving image by recording captured three-dimensional X-ray images of the ultrasound catheter, and is displayed in composite with the three-dimensional X-ray images taken during the medical procedure.

5. The X-ray diagnosis apparatus according to claim 1, wherein:
the number of X-ray detectors used is two, and
the X-ray detectors and the two X-ray tubes are configured such that one of the X-ray detectors detects the X-ray beams projected from one of the X-ray tubes and a second X-ray detector detects the X-ray beams projected from a second X-ray tube.

6. The X-ray diagnosis apparatus according to claim 1, wherein
the X-ray image capturer comprises two support arms on which one X-ray tube is provided at one end in the length direction and one X-ray detector is provided to a second end in the length direction facing the one X-ray tube; and wherein
the image capture angle changing means simultaneously rotates the two support arms in a uniform direction and to a uniform angle, and also simultaneously displaces the support arms a uniform distance to a uniform side in the length direction.

7. The X-ray diagnosis apparatus according to claim 1, wherein the three-dimensional image compositer comprises a three-dimensional image composer employing a color display as the 3D display, the compositer displaying the three-dimensional vascular moving images and the three-dimensional X-ray images in different colors on the 3D display screen, and additionally displaying blood vessels having different temporal axes in the three-dimensional vascular moving images on the 3D display screen in different colors.
